# EUROPEAN PATENT APPLICATION

(11) **EP 2 253 313 A1**
(43) Date of publication of application: **24.11.2010**
(21) Application number: 10007472.3
(22) Date of filing: 12.01.2006
(51) Int. Cl.: A61K 31/196, A61P 25/02, A61P 37/02

(54) **Tranilast as modulator of T cell functioning for use in the treatment of autoimmune diseases**

(30) Priority: 14.01.2005 US 644502 P
(62) Divisional of application: 06718328.5
(71) Applicant: The Board Of Trustees Of The University Of the Leland Stanford Junior University, Palo Alto, CA 94306-1106 (US)
(72) Inventor: Steinman, Lawrence, Stanford, California 94305 (US); Platten, M., 72076 Tuebingen (DE); Ho, Peggy Pui-Kay, Cupertino, CA 95014 (US); Selley, Michael L., Sydney, NSW 2000 (AU)
(74) Representative: Harrison, Susan Joan

(57) **Abstract**

A method of modulating T_{H}l cell functioning in a mammal, comprising administering to said mammal an effective amount of one or more IDO-mediated tryptophan metabolites or derivatives thereof, or an antagonist thereof.

## Description

### FIELD OF THE INVENTION

The present invention relates generally to a method of modulating cellular functioning and agents useful for same. More particularly, the present invention relates to a method of modulating T_{H}1 cell functioning utilising a tryptophan metabolite or derivative thereof, such as a compound of formula (I). The method of the present invention is useful, *inter alia,* in the treatment and/or prophylaxis of conditions characterised by aberrant, unwanted or otherwise inappropriate T_{H}1 cell functioning, in particular autoimmune T_{H}1 functioning, such as multiple sclerosis, by skewing the autoreactive T_{H}1 response towards a T_{H}2 response.

### BACKGROUND OF THE INVENTION

Bibliographic details of the publications referred to by author in this specification are collected alphabetically at the end of the description.

"Autoimmune disease" describes the group of illnesses in which the immune system becomes misdirected and attacks one or more of the organs which it was actually designed to protect. About 75% of autoimmune disease occurs in women, most frequently during the childbearing years.

The immune system is a complicated network of cells and cell components that normally work to defend the body and eliminate infections caused by bacteria, viruses, and other invading microbes. Where a person has an autoimmune disease, the immune system mistakenly attacks self, targeting the cells, tissues, and organs of a person's own body. A collection of immune system cells and molecules at a target site is broadly referred to as inflammation.

There are many different types of autoimmune diseases, and they can each affect the body in different ways. For example, the autoimmune reaction is directed to the myelin in multiple sclerosis and the gut in Crohn's disease. In other autoimmune diseases such as systemic lupus erythematosus (lupus), affected tissues and organs may vary among individuals with the disease. One person with lupus may have affected skin and joints whereas another may have affected skin, kidney, and lungs. Ultimately, damage to certain tissues by the immune system may be permanent, as with destruction of insulin-producing cells of the pancreas in Type 1 diabetes mellitus.

The triggers for autoimmune diseases are diverse and include immunological, genetic, viral, drug-induced and hormonal factors, acting singly or in combination. At present many individual mechanisms have been identified, but how they interact with the immune network to induce such an aberrant response is likely to vary from one situation or disease condition to the next and largely has not been elucidated. Mechanisms that have been shown to eventually cause a breakdown of self tolerance include:
(1) infection of somatic tissue by viruses,
(2) development of altered self-Ags due to binding of certain drugs to cell surfaces,
(3) cross reactivity of some Abs to bacterial Ags and self-determinants,
(4) development of newly exposed Ags in the body,
(5) the influence of hormones, and
(6) breakdown in the immune network that recognizes self.
Autoimmune diseases are often chronic, requiring lifelong care and monitoring. Currently, few autoimmune diseases can be cured.

Multiple sclerosis (MS) is a neurological autoimmune disease characterised by demyelinated lesions in the central nervous system associated with axonal damage and neuronal loss. Clinical manifestations include visual loss, extra-ocular movement disorders, paresthesias, loss of sensation, weakness, dysarthria, spasticity, ataxia, and bladder dysfunction. The usual pattern is one of recurrent attacks followed by partial recovery, but acute fulminating and chronic progressive forms also occur. As remyelination and neuronal loss cannot by spontaneously repaired, the damage caused by the autoimmune attack results in permanent neurological impairment that can worsen with disease progression.

Accordingly, there is an ongoing need to develop novel means of treating diseases, such as autoimmune diseases, which are characterised by aberrant immune cell functioning. The development of therapeutic and/or prophylactic treatment regimes which provide an alternative to steroid and immunosuppression based treatments would be highly valuable when considered in light of the seriousness of the side-effects which can be associated with these current treatments.

Tryptophan plays a unique role in defence against infection because of its relative scarcity compared to other amino acids. During infection, the body induces tryptophan-catabolizing enzymes which increase tryptophan's scarcity in an attempt to starve the infecting organisms [Brown, *et al.,* 1991]. In unresolved chronic infections, tryptophan metabolism remains disturbed. The biological disturbances caused by widespread tryptophan deficiency may be substantially responsible for some of the cognitive deficits, neuroendocrine dysregulation, and immune incompetence associated with AIDS, autoimmune disease, and other chronic disease states.

Tryptophan is metabolized in several tissues by different enzyme systems. The primary site of tryptophan catabolism is the liver where tryptophan oxidase metabolizes tryptophan with molecular oxygen as the oxidizing agent. The oxygen is used to split the 5-member nitrogen-containing ring on the tryptophan molecule generating kynurenine (KYN) derivatives.

A little over a decade ago, tryptophan oxidase was widely believed to be the only tryptophan-catabolizing enzyme. Then Japanese researchers discovered indoleamine-2,3-dioxygenase (IDO), also called indole oxidase. In peripheral tissues and in the brain, IDO is the only tryptophan-catabolizing enzyme, using superoxide anion as the oxidizing agent. IDO is a more general enzyme. It has a limited capacity to oxidize a broad class of compounds called indoles which are chemically related to tryptophan. IDO has less specificity for tryptophan than the hepatic tryptophan oxidase enzyme.

In work leading up to the present invention, it has been determined that the tryptophan metabolites generated by the IDO enzyme system, and derivatives thereof such as tranilast, down-regulate the functioning of T_{H}1 cells in the context of skewing a T_{H}1 response towards a T_{H}2 response. These findings are of great significance since the elucidation of means to downregulate T_{H}1 cell functioning provides means for selectively regulating T_{H}1 cell immune responses, in particular autoimmune conditions such as demyelination conditions. Accordingly, the present invention now provides a powerful means of selectively downregulating T_{H}1 cell functioning in a manner which avoids the side effects associated with conventional immunosuppression, this conventional form of immunosuppression being directed to downregulating the functioning of all immune cells.

### SUMMARY OF THE INVENTION

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

The subject specification contains nucleotide and amino acid sequence information prepared using the programme PatentIn Version 3.1, presented herein after the bibliography.

Each nucleotide sequence is identified in the sequence listing by the numeric indicator <210> followed by the sequence identifier (eg. <210>1, <210>2, etc). The length, type of sequence (DNA, amino acid etc) and source organism for each nucleotide sequence is indicated by information provided in the numeric indicator fields <211>, <212> and <213>, respectively. Nucleotide and amino acid sequences referred to in the specification are identified by the indicator SEQ ID NO: followed by the sequence identifier (eg. SEQ ID NO:1, SEQ ID NO:2, etc.). The sequence identifier referred to in the specification correlates to the information provided in numeric indicator field <400> in the sequence listing, which is followed by the sequence identifier (eg. <400>1, <400>2, etc). That is SEQ ID NO:1 as detailed in the specification correlates to the sequence indicated as <400>1 in the sequence listing

One aspect of the present invention is directed to a method of down-regulating T_{H}1 cell functioning in a mammal, said method comprising administering to said mammal an effective amount of one or more IDO-mediated tryptophan metabolites or derivatives thereof.

In another aspect, there is provided a method of down-regulating autoimmune T_{H}1 cell functioning in a mammal, said method comprising administering to said mammal an effective amount of one or more IDO-mediated tryptophan metabolites or derivatives thereof for a time and under conditions sufficient to skew a T_{H}1 cell response to a T_{H}2 cell response.

In a preferred embodiment, the IDO-mediated tryptophan metabolite or derivative thereof is a compound of formula (I): 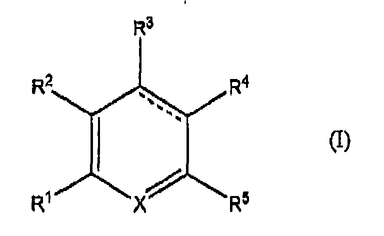 wherein
X is selected from N and CR⁶;
----- represents a single or double bond;
R¹ is selected from H, C₁₋₄alkyl, OH, C₁₋₄alkoxy, halo, CO₂H and CO₂C₁₋₄alkyl;
R² is selected from H, C₁-₄alkyl, OH, C₁₋₄alkoxy, halo, or R¹ and R² together form an optionally substituted fused phenyl ring;
R³ is selected from H, C₁₋₄alkyl, OH, C₁₋₄alkoxy and halo;
R⁴ is selected from H, C₁₋₄alkyl, C₂₋₄alkenyl, OH, C₁₋₄alkoxy, CO₂H, CO₂C₁₋₄alkyl and 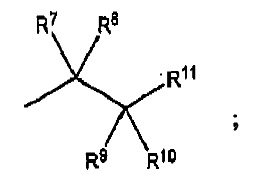 R⁵ is selected from C₁₋₄alkyl, OH, C₁₋₄alkoxy, halo, CO₂H, CO₂C₁₋₄alkyl, NH₂ and NHR¹²;
R⁶ is selected from H, C₁₋₄alkyl, OH and C₁₋₄alkoxy;
R⁷, R⁸, R⁹ and R¹⁰ are each independently H and C₁₋₄alkyl or R⁷ and R⁸ together form an oxo group or R⁷ and R⁹ form a bond;
R¹¹ is selected from CH(CO₂H)NH₂, CH(CO₂C₁₋₄alkyl)NH₂, C(O)CO₂H, C(O)CO₂C₁₋₄alkyl, C(O)H, CO₂H, CO₂C₁₋₄alkyl, C(O)NH₂, C(O)NHR¹³, CH₃NH₂, CH₂NHC₁₋₄alkyl and CH₂N(C₁₋₄alkyl)₂;
R¹² is selected from H, C₁₋₄alkyl and C(O)H; and
R¹³ is H, C₁₋₄alkyl and optionally substituted phenyl, wherein optionally substituted phenyl is optionally substituted with one or more, C₁₋₄alkyl, OH, C₁₋₄alkoxy, CO₂H, CO₂C₁₋₄alkyl, halo, NH₂, NHC₁₋₄alkyl and N(C₁₋₄alkyl)₂.

In one preferred embodiment said IDO-mediated tryptophan metabolite is 3-hydroxykynurenic acid (3-HKA), 3-hydroxyanthranilic acid (3-HAA), picolinic acid (PA) or quinolinic acid (QA).

In another preferred embodiment, said IDO-mediated tryptophan metabolite derivative is a compound of formula (II): 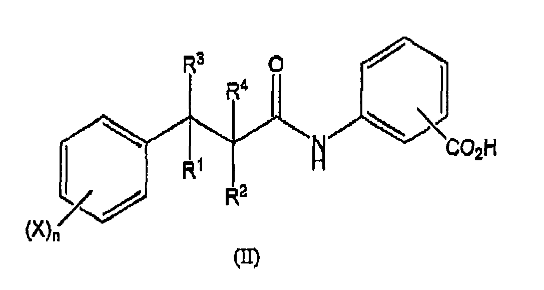 wherein each of R¹ and R² is independently selected from a hydrogen atom or a C₁-C₄alkyl group, R³ and R⁴ are each hydrogen atoms or together form another chemical bond, each X is independently selected from a hydroxyl group, a halogen atom, a C₁-C₄alkyl group or a C₁-C₄alkoxy group, or when two X groups are alkyl or alkoxy groups, they may be connected together to form a ring, and n is an integer from 1 to 3.

The carboxyl group may be in the 2-, 3- or 4-position of the aromatic ring. Preferably the carboxyl group is in the 2-position.

Preferably at least one of R¹ and R² is a hydrogen atom. More preferably, both of R¹ and R² are hydrogen atoms.

Preferably R³ and R⁴ taken together form a chemical bond. Such compounds having an unsaturated bond may be in the form of E or Z geometric isomers.

Preferably n is 1 or 2 and each X, which may be the same or different, is selected from halogen, C₁-C₄ alkyl or C₁-C₄alkoxy. Preferably X is selected from halogen and C₁-C₄alkoxy. More preferably, n is 2 and both X are selected from C₁-C₄alkoxy, especially when both X are methoxy.

Particularly preferred compounds useful in the invention are those of formula (II):

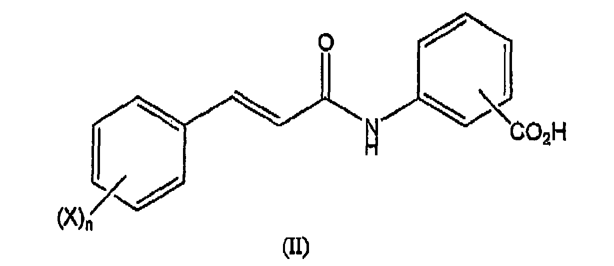

Examples of compounds of formula (II) include
2-[[3-(2-methytphenyl)-1-oxo-2-propenyl]amino]benzoic acid;
2-[[3-(3-methylphenyl)-1-oxo-2-propenyl]amino]benzoic acid;
2-[[3-(4-methylphenyl)-1-oxo-2-propenyl]amino]benzoic acid;
2-[[3-(2-etylphenyl)-1-oxo-2-propenyl]amino]benzoic acid;
2-[[3-(3-ethylphenyl)-1-oxo-2-propenyl]amino]benzoic acid;
2-[[3-(4-ethylphenyl)-1-oxo-2-propenyl]amino]benzoic acid;
2-[[3-(2-propylphenyl)-1-oxo-2-propenyl]amino]benzoic acid;
2-[[3-(3-propylphenyl)-1-oxo-2-propenyl]amino]benzoic acid;
2-[[3-(4-propylphenyl)-1-oxo-2-propenyl]amino]benzoic acid;
2-[[3-(2-hydroxyphenyl)-1-oxo-2-propenyl]amino]benzoic acid;
2-[[3-(3-hydroxyphenyl)-1-oxo-2-propenyl]amino]benzoic acid;
2-[[3-(4-hydroxyphenyl)-1-oxo-2-propenyl]amino]benzoic acid;
2-[[3-(2-chlorophenyl)-1-oxo-2-propenyl]amino]benzoic acid;
2-[[3-(3-chlorophenyl)-1-oxo-2-propenyl]amino]benzoic acid;
2-[[3-(4-chlorophenyl)-1-oxo-2-propenyl]amino]benzoic acid;
2-[[3-(2-fluorophenyl)-1-oxo-2-propenyl]amino]benzoic acid;
2-[[3-(3-fluorophenyl)-1-oxo-2-propenyl]amino]benzoic acid;
2-[[3-(4-fluorophenyl)-1-oxo-2-propenyl]amino]benzoic acid;
2-[[3-(2-bromophenyl)-1-oxo-2-propenyl]amino]benzoic acid;
2-[[3-(3-bromophenyl)-1-oxo-2-propenyl]amino]benzoic acid;
2-[[3-(4-bromophenyl)-1-oxo-2-propenyl]amino]benzoic acid;
2-[[3-(2,3-dimethoxyphenyl)-1-oxo-2-propenyl]amino]benzoic acid;
2-[[3-(3,4-dimethoxyphenyl)-1-oxo-2-propenyl]amino]benzoic acid;
2-[[3-(2,4-dimethoxyphenyl)-1-oxo-2-propenyl]amino]benzoic acid;
2-[[3-(2,3-dimethylphenyl)-1-oxo-2-propenyl]amino]benzoic acid;
2-[[3-(3,4-dimethylphenyl)-1-oxo-2-propenyl]amino]benzoic acid;
2-[[3-(2,4-dimethylphenyl)-1-oxo-2-propenyl]amino]benzoic acid;
2-[[3-(2,3-diethoxyphenyl)-1-oxo-2-propenyl]amino]benzoic acid;
2-[[3-(3,4-diethoxyphenyl)-1-oxo-2-propenyl]amino]benzoic acid;
2-[[3-(2,4-diethoxyphenyl)-1-oxo-2-propenyl]amino]benzoic acid;
2-[[3-(2,3-dipropoxyphenyl)-1-oxo-2-propenyl]amino]benzoic acid;
2-[[3-(3,4-dipropoxyphenyl)-1-oxo-2-propenyl]amino]benzoic acid;
2-[[3-(2,4-dipropoxyphenyl)-1-oxo-2-propenyl]amino]benzoic acid;
2-[[3-(2,3-diethylphenyl)-1-oxo-2-propenyl]amino]benzoic acid;
2-[[3-(3,4-diethylphenyl)-1-oxo-2-propenyl]amino]benzoic acid;
2-[[3-(2,4-diethylphenyl)-1-oxo-2-propenyl]amino]benzoic acid;
2-[[3-(2,3-dipropylphenyl)-1-oxo-2-propenyl]amino]benzoic acid;
2-[[3-(3,4-dipropylphenyl)-1-oxo-2-propenyl]amino]benzoic acid;
2-[[3-(2,4-dipropylphenyl)-1-oxo-2-propenyl]amino]benzoic acid;
2-[[3-(2-methoxy-3-methylphenyl)-1-oxo-2-propenyl]amino]benzoic acid;
2-[[3-(3-methoxy-4-methylphenyl)-1-oxo-2-propenyl]amino]benzoic acid;
2-[[3-(2-methoxy-3-methylphenyl)-1-oxo-2-propenyl]amino]benzoic acid;
2-[[3-(2-methoxy-4-methylphenyl)-1-oxo-2-propenyl]amino]benzoic acid;
2-[[3-(2-methoxy-3-chlorophenyl)-1-oxo-2-propenyl]amino]benzoic acid;
2-[[3-(3-methoxy-4-chlorophenyl)-1-oxo-2-propenyl]amino]benzoic acid;
2-[[3-(2-methoxy-3-chlorophenyl)-1-oxo-2-propenyl]amino]bebzoic acid;
2-[[3-(2-methoxy-4-chlorophenyl)-1-oxo-2-propenyl]amino]benzoio acid;
2-[[3-(2-methoxy-3-hydroxyphenyl)-1-oxo-2-propenyl]amino]benzoic acid;
2-[[3-(3-methoxy-4-hydroxyphenyl)-1-oxo-2-propenyl]amnio]benzoic acid;
2-[[3-(2-methoxy-3-hydroxyphenyl)-1-oxo-2-propenyl]amino]benzoic acid;
2-[[3-(2-methoxy-4-hydroxyphenyl)-1-oxo-2-propenyl]amino]benzoic acid;
2-[[3-(3,4-trimethylenephenyl)-1-oxo-2-propenyl]amino]benzoic acid;
2-[[3-(2,3-trimethylenephenyl)-1-oxo-2-propenyl]amino]benzoic acid;
2-[[3-(3,4-methylenedioxyphenyl)-1-oxo-2-propenyl]amino]benzoic acid; and
2-[[3-(3,4-ethylenedioxyphenyl)-1-oxo-2-propenyl]amino]benzoic acid.

A particularly preferred compound of formula (II) for use in the invention is 2-[[3-(3,4-dimethoxyphenyl)-1-oxo-2-propenyl]amino]benzoic acid (tranilast, TNL).

In yet another aspect there is provided a method of down-regulating autoimmune T_{H}1 cell functioning in a mammal, said method comprising administering to said mammal an effective amount of one or more IDO-mediated tryptophan metabolites or derivatives thereof for a time and under conditions sufficient to skew the subject T_{H}1 cell response to a T_{H}2 cell response where said metabolite or derivative thereof up-regulates T_{H}2 cytokine production.

In still another aspect there is provided a method of down-regulating autoimmune T_{H}1 cell functioning, which autoimmune T_{H}1 cell is directed to a myelin protein, in a mammal, said method comprising administering to said mammal an effective amount of one or more IDO-mediated tryptophan metabolites or derivatives thereof for a time and under conditions sufficient to skew the subject T_{H}1 cell response to a T_{H}2 cell response, wherein said metabolite or derivative thereof up-regulates T_{H}2 cytokine production.

A further aspect of the present invention is directed to a method of upregulating, in a mammal, inhibited T_{H}1 cell functioning, said method comprising administering to said mammal an effective amount of an antagonist of an IDO-mediated tryptophan metabolite or compound of formula (I) or formula (II) or a pharmaceutically acceptable salt thereof.

Another further aspect of the present invention is directed to a method for the treatment and/or prophylaxis of a condition characterised by aberrant T_{H}1 cell functioning in a mammal, said method comprising administering to said mammal an effective amount of one or more IDO-mediated tryptophan metabolites or derivatives thereof for a time and under conditions sufficient to down-regulate said T_{H}1 functioning.

In yet another further aspect there is provided a method for the treatment and/or prophylaxis of a condition characterised by autoimmune T_{H}1 cell functioning in a mammal, said method comprising administering to said mammal an effective amount of one or more IDO-modiated tryptophan metabolites or derivatives thereof for a time and under conditions sufficient to skew a T_{H}1 cell response to a T_{H}2 cell response.

In still another further aspect there is provided a method for the treatment and/or prophylaxis of a condition characterised by autoimmune T_{H}1 cell functioning in a mammal, which autoimmune T_{H}1 cell is directed to myelin basic protein, said method comprising administering to said mammal an effective amount of one or more IDO-mediated tryptophan metabolites or derivatives thereof for a time and under conditions sufficient to skew a T_{H}1 cell response to a T_{H}2 cell response wherein said metabolite or derivative thereof up-regulates T_{H}2 cytokine production.

Yet another aspect of the present invention is directed to the use an IDO-mediated tryptophan metabolite or derivative thereof in the manufacture of a medicament for the treatment of a condition characterised by aberrant T_{H}1 cell functioning wherein administering said compound down-regulates said T_{H}1 cell functioning.

Yet another aspect of the present invention is directed to the use of an IDO-mediated tryptophan metabolite or derivative thereof in the manufacture of a medicament for the treatment of multiple sclerosis.

Yet another aspect of the present invention relates to the metabolites or derivatives as hereinbefore defined or pharmaceutically acceptable salts thereof or antagonists thereof, as hereinbefore defined, when used in the method of the present invention.

### BRIEF DESCRIPTION OF THE DRAWINGS

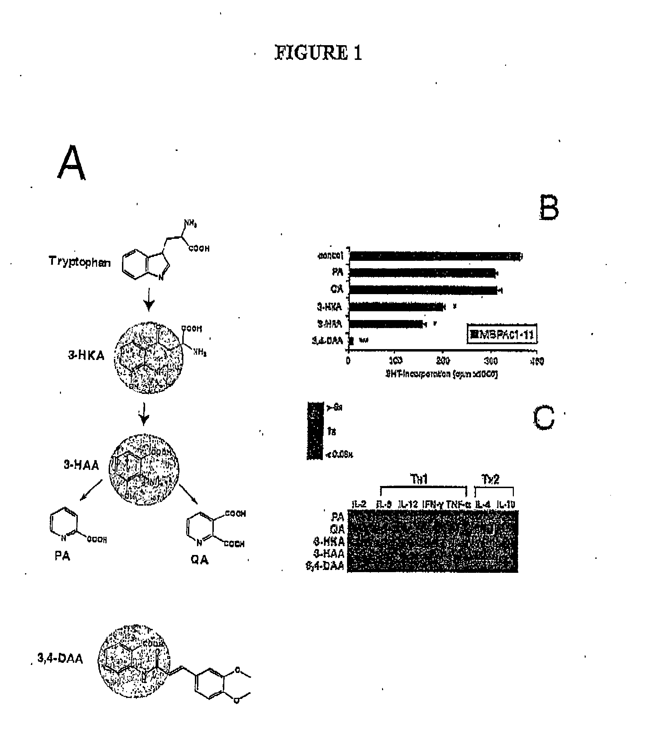

**Figures 1A-1C****:** Modulation of T cell proliferation by Trp metabolites **(A)** Chemical structure of Trp metabolites. 3-HKA, 3-hydroxy-kynurenic acid; 3-HAA, 3-hydroxy-anthranilic acid; PA, picolinic acid; QA, quinolinic acid; 3,4-DAA, N-(3,4,-dimethoxycinnamoyl)anthranilic acid. **(B)** Splenocytes from MBPAc1-11 TCR transgenic mice were incubated with Trp metabolites PA, QA, 3-HKA, 3-HAA, 3,4-DAA (200 µM) and stimulated with MBPAc1-11 (5 µg/ml). To assess proliferation cells were pulsed with ³H-thymidine after 48h for 18h. Data represent mean counts per minute (cpm) and SEM of triplicates and are representative of 4 independent experiments. *p<0.05, ***p<0.001. (C) Splenocytes from MBPAc1-11 TCR transgenic mice were activated with MBPAc1-11 (0.5 - 2.5µg/ml) in the absence or presence of Trp metabolites PA, QA, 3-HKA, 3-HAA, 3,4-DAA at 200 µM (IL-2, IFN-γ, TNF-α, IL-6 and IL-12/23 p40) or 30 µM (IL-4, IL-10). Cytokine release was measured after 48h (IL-2, IL-6, IL-12/23 p40), 72h (IFN-γ, TNF-α) or 120h (IL-4, IL-10) using ELISA (OptEIA Cytokine Sets, BD Pharmingen). Data are displayed as a heatmap.

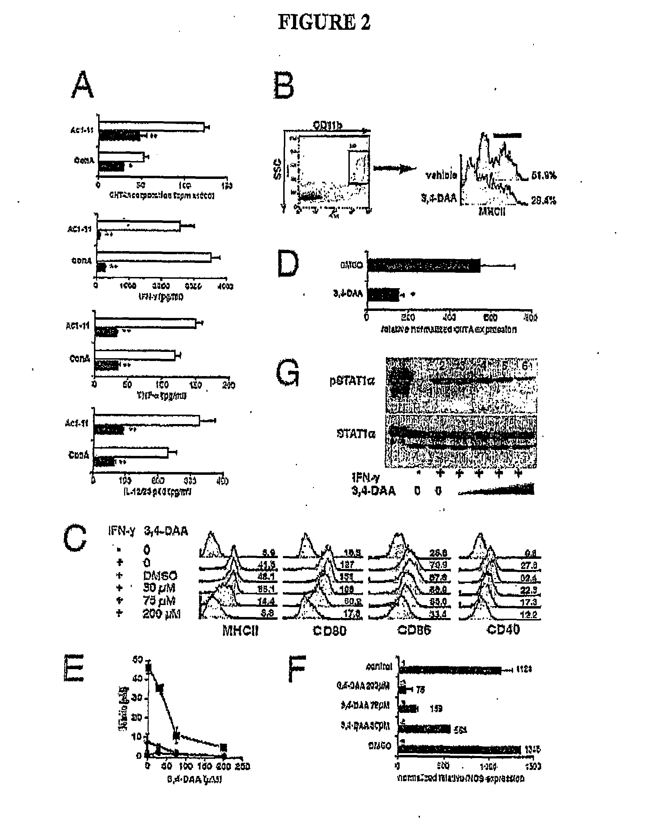

**Figures 2A-2G****:** Mechanisms of APC and T cell function modulated by 3,4-DAA. **(A, B)** MBPAo1-11 TCR transgenic mice (n=3 per group) were fed with 3,4-DAA for 5 days (500 mg/kg/d). (**A**) Pooled splenocytes of vehicle (Na-CMC)-treated (open bars) or 3,4-DAA-treated (filled bars) mice were stimulated with MBPAc1-11 (5 µg/ml) or ConA (2 µg/ml) *in vitro.* Proliferation and cytokine analysis was performed as in Fig. 1. Mean values and SEM of trip+licates are given and data are representative of 2 independent experiments. *p<0.05, **p<0.01, **(B)** Pooled splenocytes were analyzed for cell surface expression of CD11b and MHC class II (I-A^{k}) using flow cytometry. The right panel represents histograms of CD11b⁺ monocytes stained with anti-MHCII. Values represent percentages of MHC class II⁺ CD11b⁺ cells. Data are representative of 2 independent experiments. **(C-G)** EOC20 cells were incubated with media alone, vehicle (DMSO) or 3,4-DAA at the concentrations indicated and stimulated with IFN-γ (200 U/ml) and/or LPS (200 ng/ml). **(C)** Cell surface expression of MHC class II (I-A^{k}), CD40, CD80 and CD86 was determined after 48h using flow cytometry. Histograms are representative of 3 independent experiments, values represent mean fluorescent indices. **(D)** RNA was extracted after 24h and reverse transcribed. CIITA cDNA expression was semiquantified using real-timed PCR. Values represent mean arbitrary expression levels of triplicates and SEM normalized to expression of β-actin. Data are representative of 2 independent experiments. *p<0.05. **(E)** Nitrite release of unstimulated (diamonds), IFN-γ-stimulated (circles) or IFN-γ- and LPS-stimulated cells was determined after 48h using the Griess assay. Values are mean nitrite concentration and SEM of triplicates and are representative of 3 independent experiments. **(F)** RNA was extracted after 24h and reverse transcribed. iNOS cDNA expression was semiquantified using real-timed PCR. Values of unstimulated (open bars) and IFN-γ-stimulated (filled bars) represent mean arbitrary expression levels of triplicates and SEM normalized to expression of β-actin. Data are representative of 2 independent experiments. **(G)** Western blot analysis of whole cell protein extracted 15 min after stimulation with IFN-γ using a phospho-specific STAT1α antibody. The membrane was reprobed with a non-phospho-specific STAT1α antibody to ensure equal loading.

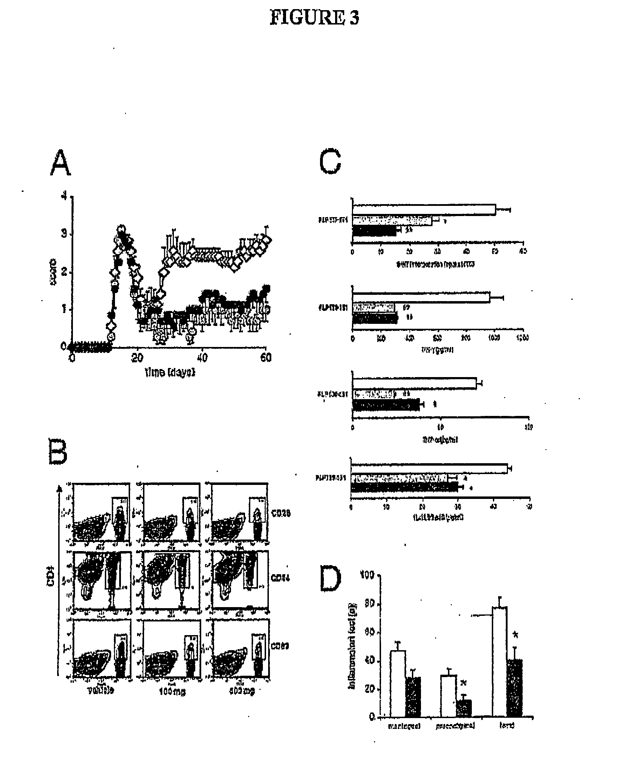

**Figures 3A-3D****:** 3,4-DAA ameliorates established EAE. **(A-D)** 7-8 week-old female SJ/L mice were immunized with PLP139-151. Treatment was initiated at d16 by oral gavage twice daily. **(A)** clinical scores of vehicle-treated mice (open diamonds) and mice treated with 3,4-DAA at 100 mg/kg/d (grey circles) or 300 mg/kg/d (black circles) (0, asymptomatic; 1, limp tail; 2, partial hind limb paresis; 3, hind limp paralysis; 4, quadriplegia; 5, moribund or dead) were assessed each day. Data represent mean scores (n=9, vehicle and 300 mg/kg/d; n=10, 100 mg/kg/day) and SEM. **(B)** flow cytometric analysis of pooled splenooytes of vehicle-treated (n=6), or 3,4-DAA-treated (n=7, 100 mg/kg/d; n=6, 300 mg/kg/d) mice isolated 60 days post immunizations. Values represent double positive cells. **(C)** Pooled splenocytes of vehicle-treated (n=6, open bars), or 3,4-DAA-treated (n=7, 100 mg/kg/d, grey bars; n=6, 300 mg/kg/d, black bars) were isolated after 60 days and stimulated in vitro with PLP139-151 (20 µg/ml). Proliferation was assessed as in Figure 3, except cells were pulsed after 72h of culture. Cytokines were analyzed as in Figure 3. Data represent mean values of triplicates and SEM. *p<0.05, **p<0.01. **(D)** Brains and spinal cords were extracted 60 days after immunization. Infiltration of inflammatory cells in randomly chosen brains from vehicle-treated (n=3, open bars) and 3,4-DAA-treated (n=3, 100 mg/kg/d, black bars) was counted by a neuropathologist blinded to the treatment. Data represent mean number of inflammatory foci and SEM. *p<0.05.

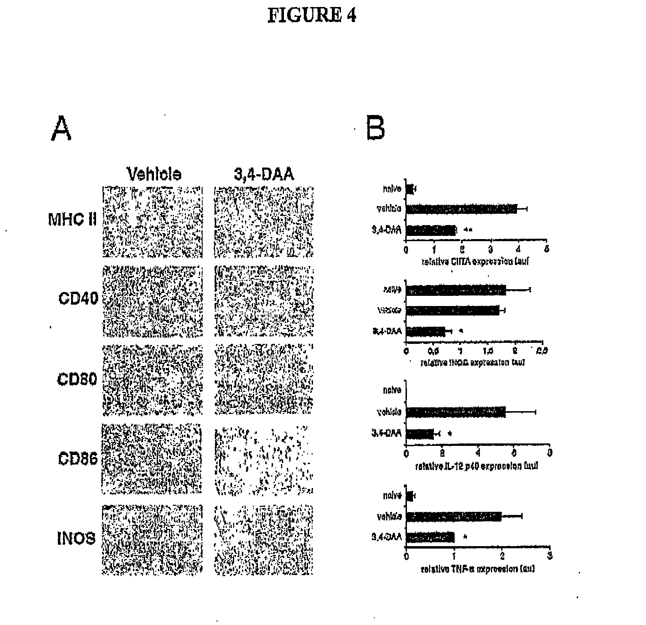

**Figures 4A-4B****:** 3,4-DAA suppresses the activation of CNS antigen-presenting cells in EAE. **(A)** 7-8 week-old female SJ/L mice were immunized with PLP₁₃₉₋₁₅₁ two days after the initiation of treatment. Brains or spinal cords were stained for MHC class II (I-A^{k}), CD40, CD80, CD86 and iNOS (**B**) 7-8 week-old female SJ/L mice were immunized with PLP₁₃₉₋₁₅₁. Treatment was initiated at d16 by oral gavage twice daily. Naïve animals served as a control. RNA was isolated from spinal cords 60 days after immunization (n=3). After reverse transcription cDNA expression of the indicated transcripts was analyzed using real-time PCR. Values represent mean arbitrary expression levels of triplicates and SEM normalized to expression of β-actin. Data are representative of 3 independent experiments. *p<0.05, **p<0.01.

### DETAILED DESCRIPTION OF THE INVENTION

The present invention is predicted, in part, on the surprising determination that IDO-mediated tryptophan metabolites and derivatives thereof down-regulate, T_{H}1 cell functioning. More specifically, the release of T_{H}1 cytokines is down-regulated concomitant with an up-regulation in the production of T_{H}2 cytokines. This necessarily results in the skewing of a T_{H} cell response from a T_{H}1 response to a T_{H}2 response thereby suppressing any further T_{H}1 responsiveness. These findings have now permitted the rational design of means for therapeutically or prophylactically treating conditions characterised by aberrant T_{H}1 cell functioning, such as autoimmune T_{H}1 cell functioning. Examples of such conditions include autoimmune demyelinating diseases such as multiple sclerosis.

Accordingly, one aspect of the present invention is directed to a method of down-regulating T_{H}1 cell functioning in a mammal, said method comprising administering to said mammal an effective amount of one or more IDO-mediated tryptophan metabolites or derivatives thereof.

More particularly, there is provided a method of down-regulating autoimmune T_{H}1 cell functioning in a mammal, said method comprising administering to said mammal an effective amount of one or more IDO-mediated tryptophan metabolites or derivatives thereof for a time and under conditions sufficient to skew a T_{H}1 cell response to a T_{H}2 cell response.

Reference to "IDO-mediated tryptophan metabolites" should be understood as a reference to any molecule which is generated pursuant to the metabolism of tryptophan via the IDO enzyme system. Examples of such metabolites include, but are not limited to, 3-Hydroxykynurenic acid (3-HKA), 3-Hydroxyanthrailic acid (3-HAA), picolinic acid (PA), and quinolinic acid (QA). The present invention should also be understood to extend to the use of derivatives of IDO-mediated tryptophan metabolites, such as tranilast. N-[3,4-dimethoxycinnamoyl]-anthranilic acid (also known as 2-[[3-(3,4-dimethoxyphenyl)-1-oxo-2-propenyl]amino]benzoic acid, tranilast, TNL) is an anti-allergic agent originally identified as an inhibitor of mast cell degranulation (Zampini P *et al.,* 1983). In accordance with the present invention, it has been determined that this molecule, which is a synthetic derivative of 3-HAA, functions to skew an autoimmune T_{H}1 response to a T_{H}2 response, thereby effectively suppressing the T_{H}1 response.

Accordingly, in one preferred embodiment said IDO-mediated tryptophan metabolite or derivative thereof is a compound of formula (I): 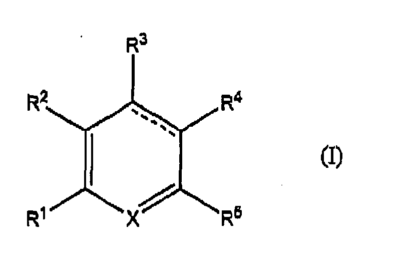 wherein
X is selected from N and CR⁶;
----- represents a single or double bond;
R¹ to selected from H, C₁₋₄alkyl, OH, C₁₋₄alkoxy, halo, CO₂H and CO₂C₁₋₄alkyl;
R² is selected from H, C₁₋₄alkyl, OH, C₁₋₄alkoxy, halo, or R¹ and R² together form an optionally substituted fused phenyl ring;
R³ is selected from H, C₁₋₄alkyl, OH, C₁₋₄alkoxy and halo;
R⁴ is selected from H, C₁₋₄alkyl, C₂₋₄alkenyl, OH, C₁₋₄alkoxy, CO₂H, CO₂C₁₋₄alkyl and 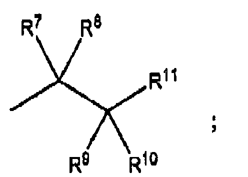 R⁵ is selected from C₁₋₄alkyl, OH, C₁₋₄alkoxy, halo, CO₂H, CO₂C₁₋₄alkyl, NH₂ and NHR¹²;
R⁶ is selected from H, C₁₋₄alkyl, OH and C₁₋₄alkoxy;
R⁷, R⁸, R⁹ and R¹⁰ are each independently H and C₁₋₄alkyl or R⁷ and R⁸ together form an oxo group or R⁷ and R⁹ form a bond;
R¹¹ is selected from CH(CO₂H)NH₂, CH(CO₂C₁₋₄alkyl)NH₂, C(O)CO₂H, C(O)CO₂C₁₋₄alkyl, C(O)H, CO₂H, CO₂C₁₋₄alkyl, C(O)NH₂, C(O)NHR¹³, CH₂NH₂, CH₂NHC₁₋₄alkyl and CH₂N(C₁₋₄alkyl)₂;
R¹² is selected from H, C₁₋₄alkyl and C(O)H; and
R¹³ is H, C₁₋₄alkyl and optionally substituted phenyl, wherein optionally substituted phenyl is optionally substituted with one or more, C₁₋₄alkyl, OH, C₁₋₄alkoxy, CO₂H, CO₂C₁₋₄alkyl, halo, NH₂, NHC₁₋₄alkyl and N(C₁₋₄alkyl)₂.

In one preferred embodiment said IDO-mediated tryptophan metabolite is 3-HKA, 3-HAA, PA or QA.

In another preferred embodiment, said IDO-mediated tryptophan metabolite derivative is a compound of formula (II): 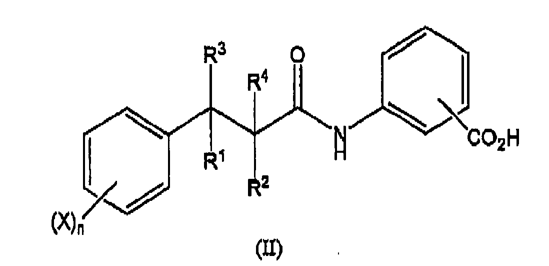 wherein each of R¹ and R² is independently selected from a hydrogen atom or a C₁-C₄alkyl group, R³ and R⁴ are each hydrogen atoms or together form another chemical bond, each X is independently selected from a hydroxyl group, a halogen atom, a C₁-C₄alkyl group or a C₁-C₄alkoxy group, or when two X groups are alkyl or alkoxy groups, they may be connected together to form a ring, and n is an integer from 1 to 3.

The carboxyl group may be in the 2-, 3- or 4-position of the aromatic ring. Preferably the carboxyl group is in the 2-position.

Preferably at least one of R¹ and R² is a hydrogen atom. More preferably, both of R¹ and R² are hydrogen atoms.

Preferably R³ and R⁴ taken together form a chemical bond. Such compounds having an unsaturated bond may be in the form of E or Z geometric isomers.

Preferably n is 1 or 2 and each X, which may be the same or different, is selected from halogen, C₁-C₄ alkyl or C₁-C₄alkoxy. Preferably X is selected from halogen and C₁-C₄alkoxy. More preferably, n is 2 and both X are selected from C₁-C₄alkoxy, especially when both X are methoxy.

Particularly preferred compounds useful in the invention are those of formula (III):

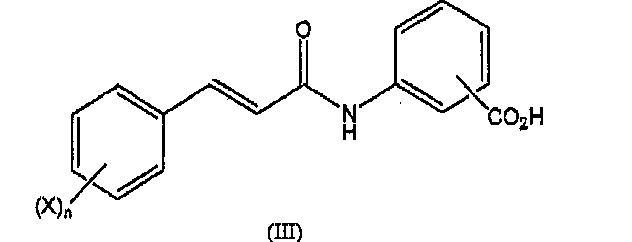

Examples of compounds of formula (II) include
2-[[3-(2-methylphenyl)-1-oxo-2-propenyl]amino]benzoic acid;
2-[[3-(3-methylphenyl)-1-oxo-2-propenyl]amino]benzoic acid;
2-[[3-(4-methylphenyl)-1-oxo-2-propenyl]amino]benzoic acid;
2-[[3-(2-ethylphenyl)-1-oxo-2-propenyl]amino]benzoic acid;
2-[[3-(3-ethylphenyl)-1-oxo-2-propenyl]amino]benzoic acid;
2-[[3-(4-ethylphenyl)-1-oxo-2-propenyl]amino]benzoic acid;
2-[[3-(2-propylphenyl)-1-oxo-2-propenyl]amino]benzoic acid;
2-[[3-(3-propylphenyl)-1-oxo-2-propenyl]amino]benzoic acid;
2-[[3-(4-propylphenyl)-1-oxo-2-propenyl]amino]benzoic acid;
2-[[3-(2-hydroxyphenyl)-1-oxo-2-propenyl]amino]benzoic acid;
2-[[3-(3-hydroxyphenyl)-1-oxo-2-propenyl]amino]benzoic acid;
2-[[3-(4-hydroxyphenyl)-1-oxo-2-propenyl]amino]benzoic acid;
2-[[3-(2-chlorophenyl)-1-oxo-2-propenyl]amino]benzoic acid;
2-[[3-(3-chlorophenyl)-1-oxo-2-propenyl]amino]benzoic acid;
2-[[3-(4-chlorophenyl)-1-oxo-2-propenyl]amino]benzoic acid;
2-[[3-(2-fluorophenyl)-1-oxo-2-propenyl]amino]benzoic acid;
2-[[3-(3-fluorophenyl)-1-oxo-2-propenyl]amino]benzoic acid;
2-[[3-(4-fluorophenyl)-1-oxo-2-propenyl]amino]benzoic acid;
2-[[3-(2-bromophenyl)-1-oxo-2-propenyl]amino]benzoic acid;
2-[[3-(3-bromophenyl)-1-oxo-2-propenyl]amino]benzoic acid;
2-[[3-(4-bromophenyl)-1-oxo-2-propenyl]amino]benzoic acid;
2-[[3-(2,3-dimethoxyphenyl)-1-oxo-2-propenyl]amino]benzoic acid;
2-[[3-(3,4-dimethoxyphenyl)-1-oxo-2-propenyl]amino]benzoic acid;
2-[[3-(2,4-dimcthoxyphenyl)-1-oxo-2-propenyl]amino]benzoic acid;
2-[[3-(2,3-dimethylphenyl)-1-oxo-2-propenyl]amino]benzoic acid;
2-[[3-(3,4-dimethylphenyl)-1-oxo-2-propenyl]amino]benzoic acid;
2-[[3-(2,4-dimethylphenyl)-1-oxo-2-propenyl]amino]benzoic acid;
2-[[3-(2,3-diethoxyphenyl)-1-oxo-2-propenyl]amino]benzoic acid;
2-[[3-(3,4-diethoxyphenyl)-1-oxo-2-propenyl]amino]benzoic acid;
2-[[3-(2,4-diethoxyphenyl)-1-oxo-2-propenyl]amino]benzoic acid;
2-[[3-(2,3-dipropoxyphenyl)-1-oxo-2-propenyl]amino]benzoic acid;
2-[[3-(3,4-dipropoxyphenyl)-1-oxo-2-propenyl]amino]benzoic acid;
2-[[3-(2,4-dipropoxyphenyl)-1-oxo-2-propenyl]amino]benzoic acid;
2-[[3-(2,3-diethylphenyl)-1-oxo-2-propenyl]amino]benzoic acid;
2-[[3-(3,4-diethylphenyl)-1-oxo-2-propenyl]amino]benzoic acid;
2-[[3-(2,4-diethylphenyl)-1-oxo-2-propenyl]amino]benzoic acid;
2-[[3-(2,3-dipropylphenyl)-1-oxo-2-propenyl]amino]benzoic acid;
2-[[3-(3,4-dipropylphenyl)-1-oxo-2-propenyl]amino]benzoic acid;
2-[[3-(2,4-dipropylphenyl)-1-oxo-2-propenyl]amino]benzoic acid;
2-[[3-(2-methoxy-3-methylphenyl)-1-oxo-2-propenyl]amino]benzoic acid;
2-[[3-(3-methoxy-4-methylphenyl)-1-oxo-2-propenyl]amino]benzoic acid;
2-[[3-(2-methoxy-3-methylphenyl)-1-oxo-2-propenyl]amino]benzoic acid;
2-[[3-(2-methoxy-4-methylphenyl)-1-oxo-2-propenyl]amino]benzoic acid;
2-[[3-(2-methoxy-3-chlorophenyl)-1-oxo-2-propenyl]amino]benzoic acid;
2-[[3-(3-methoxy-4-chlorophenyl)-1-oxo-2-propenyl]amino]benzoic acid;
2-[[3-(2-methoxy-3-chlorophenyl)-1-oxo-2-propenyl]amino]benzoic acid;
2-[[3-(2-methoxy-4-chlorophenyl)-1-oxo-2-propenyl]amino]benzoic acid;
2-[[3-(2-methoxy-3-hydroxyphenyl)-1-oxo-2-propenyl]amino]benzoic acid;
2-[[3-(3-methoxy-4-hydroxyphenyl)-1-oxo-2-propenyl]amino]benzoic acid;
2-[[3-(2-methoxy-3-hydroxyphenyl)-1-oxo-2-propenyl]amino]benzoic acid;
2-[[3-(2-methoxy-4-hydroxyphenyl)-1-oxo-2-propenyl]amino]benzoic acid;
2-[[3-(3-4-trimethylenephenyl)-1-oxo-2-propenyl]amino]benzoic acid;
2-[[3-(2,3-trimethylenephenyl)-1-oxo-2-propenyl]amino]benzoic acid;
2-[[3-(3,4-methylenedioxyphenyl)-1-oxo-2-propenyl]amino]benzoic acid; and
2-[[3-(3,4-ethylenedioxyphenyl)-1-oxo-2-propenyl]amino]benzoic acid.

A particularly preferred compound of formula (III) for use in the invention is 2-[[3-(3,4-dimethoxyphenyl)-1-oxo-2-propenyl]amino]benzoic acid (tranilast, TNL).

As used herein, the term "C₁-C₄alkyl" refers to linear or branched hydrocarbon chains having 1 to 4 carbon atoms. Examples of such groups include methyl, ethyl, n-propyl, isopropyl, n-butyl, sec-butyl and tert-butyl.

As used herein, the term "C₂-C₄alkenyl" refers to linear or branched hydrocarbon chains having 2 to 4 carbon atoms and one or two double bonds. Examples of such groups include vinyl, propenyl, butenyl and butadienyl.

As used herein, the term "C₁-C₄alkoxy" refers to hydroxy groups substituted with linear or branched alkyl groups having 1 to 4 carbon atoms. Examples of such groups include methoxy, ethoxy, n-propoxy, isopropoxy, n-butoxy, sec-butoxy and tert-butoxy.

As used herein, the term "halogen" or "halo" refers to fluoro, chloro or bromo atoms.

Suitable pharmaceutically acceptable salts include, but are not limited to, salts of pharmaceutically acceptable inorganic acids such as hydrochloric, sulphuric, phosphoric, nitric, carbonic, boric, sulfamic, and hydrobromic acids, or salts of pharmaceutically acceptable organic acids such as acetic, propionic, butyric, tartaric, maleic, hydroxymaleic, fumaric, maleic, citric, lactic, mucic, gluconic, benzoic, succinic, oxalic, phenylacetic, methanesulphonic, toluenesulphonic, benzenesulphonic, salicyclic sulphanilic, aspartic, glutamic, edetic, stearic, palmitic, oleic, lauric, pantothenic, tannic, ascorbic and valeric acids.

Base salts include, but are not limited to, those formed with pharmaceutically acceptable cations, such as sodium, potassium, lithium, calcium, magnesium, ammonium and alkylammonium.

Basic nitrogen-containing groups may be quarternised with such agents as lower alkyl halide, such as methyl, ethyl, propyl, and butyl chlorides, bromides and iodides; dialkyl sulfates like dimethyl and diethyl sulfate; and others.

Compounds of formula (I) and their pharmaceutically acceptable salts are known and may be prepared by methods known in the art, see US 3,940,422 the contents of which are incorporated herein by reference.

It will also be recognised that some compounds of formula (I) may possess asymmetric centres and are therefore capable of existing in more than one stereoisomeric form. The invention thus also relates to compounds in substantially pure isomeric form at one or more asymmetric centres eg., greater than about 90% ee, such as about 95% or 97% ee or greater than 99% ee, as well as mixtures, including racemic mixtures, thereof. Such isomers may be prepared by asymmetric synthesis, for example using chiral intermediates or by chiral resolution.

Without limiting the present invention to any one theory or mode of action, the compounds of formula (I) are orally active anti-allergic compounds. A particularly preferred compound of the invention is known by either of the chemical names N-[3,4-dimethoxycinnamoyl]-anthranilic acid or 2-[[3-(3,4-dimethoxyphenyl)-1-oxo-2-propenyl]amino]benzoic acid and may also be referred to as Tranilast. Still further, it is known by the chemical formula C₁₈H₁₇NO₅ and by the trade name Rizaben. The structure of N-[3,4-dimethoxyoinnamoyl]-anthranilic acid is depicted below:

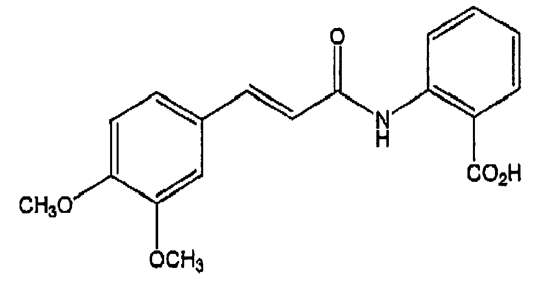

Reference to a "T_{H}1 cell" or a "T_{H}2 cell" should be understood as a reference to the immune cells which express a T cell receptor together with CD4 and which act as an inducer of the effector cell for the humoral immune response or cell-mediated immumty/inflammation. Without limiting the present invention to any one theory or mode of action, these cells recognise and bind to antigen which is presented in the context of MHC Class II molecules expressed on the surface of antigen presenting cells. More specifically, T_{H}1 cells are functionally defined as T_{H} cells which produce, *inter alia,* IL-2, IFN-γ, and TNF-α and mediate cell mediated/inflammatory immune responses. T_{H}2 cells are functionally defined as T_{H} cells which produce, *inter alia,* IL-4, IL-5 and IL-10 and mediate the humoral response. There occurs cross inhibition of these T_{H} subclasses in that production of the cytokines characteristic of any one subclass promotes the expansion and functioning of the T_{H} cells of that subclass while down-regulating the functioning of the other subclass.

Still without limiting the present invention in any way, in autoimmune diseases such as multiple sclerosis, T_{H}1-differentiated autoreactive CD4⁺ cells are driving the inflammatory process. In fact, therapeutic approaches aim at skewing the cyokine profile of myelin-specific autoimmune T_{H} cells from T_{H}1 to T_{H}2 such as via the use of altered peptide ligands (APL), HMG-CoA reductase inhibitors ("Statins") or DNA vaccination combined with gene delivery of IL-4. These have been shown to be successful in animal models of multiple sclerosis (Brocke, S, et al., Nature 379, 343-6, 1996; Garren, H., et al., Immunity 15, 15-22, 2001; Youssef, S., et al., Nature 420, 78-84, 2002). Moreover, currently approved treatments for multiple sclerosis have been shown to induce a T_{H}2 shift in the cytokine profile of patients with multiple sclerosis (Steinman, L., Science 305, 212-6, 2004). Reference to "T cell" should also be understood to encompass reference to T cell mutants. "Mutants" include, but are not limited to T cells which have been naturally or non-naturally modified, such as cells which are genetically modified. Reference to "T cells" should also be understood to extend to cells which exhibit commitment to the T cell image. These cells may be at any differentiative stage of development.

Reference to T_{H}1 "functioning" should be understood as a reference to any one or more of the functional activities which a T_{H}1 cell at any differentiative stage of development, is capable of performing. This includes, for example, T_{H}1 proliferation, differentiative and/or cytokine production. It should also be understood to extend to the up-regulation of T_{H}2 functioning which, due to the cross-inhibition of the subclasses, effectively down-regulates T_{H}1 functioning. Preferably, said T_{H}1 functioning is down-regulated via up-regulation of the production of T_{H}2 cytokines.

According to this preferred embodiment, there is provided a method of down-regulating autoimmune T_{H}1 cell functioning in a mammal, said method comprising administering to said mammal an effective amount of one or more IDO-mediated tryptophan metabolites or derivatives thereof for a time and under conditions sufficient to skew the subject T_{H}1 cell response to a T_{H}2 cell response where said metabolite or derivative thereof up-regulates T_{H}2 cytokine production.

Reference to the subject T_{H}1 cell being an "autoimmune" cell should be understood to mean that the T cell receptor (TCR) of said T_{H}1 cell is directed to a self antigen. In this regard, the T_{H}1 cell TCR may be uniquely and exclusively directed to a self antigen, it may be directed to a non-self antigen but nevertheless exhibits cross-reactivity with a self antigen or it may be directed to a self antigen but nevertheless exhibit cross-reactivity with a non-self antigen. Without limiting the present invention to any one theory or mode of action, the activation and induction of effector functions of a T cell by a self antigen corresponds to an autoimmune response. Preferably, the subject autoantigen is a myelin protein and even more preferably the myelin basic protein.

There is therefore still more preferably provided a method of down-regulating autoimmune T_{H}1 cell functioning, which autoimmune T_{H}1 cell is directed to a myelin protein, in a mammal, said method comprising administering to said mammal an effective amount of one or more IDO-mediated tryptophan metabolites or derivatives thereof for a time and under conditions sufficient to skew the subject T_{H}1 cell response to a T_{H}2 cell response, wherein said metabolite or derivative thereof up-regulates T_{H}2 cytokine production.

Preferably, said myelin protein is myelin basic protein.

In a preferred embodiment, the IDO-mediated tryptophan metabolite or derivative thereof is a compound of formula (I): 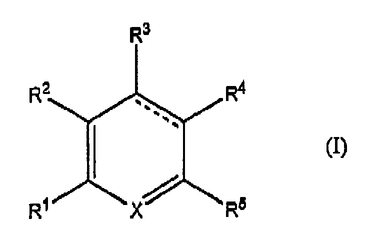 wherein
X is selected from N and CR⁶;
----- represents a single or double bond;
R¹ is selected from H, C₁-₄alkyl, OH, C₁₋₄alkoxy, halo, CO₂H and CO₂C₁₋₄alkyl;
R² is selected from H, C₁₋₄alkyl, OH, C₁₋₄alkoxy, halo, or R¹ and R² together form an optionally substituted fused phenyl ring;
R³ is selected from H, C₁₋₄alkyl, OH, C₁₋₄alkoxy and halo;
R⁴ is selected from H, C₁₋₄alkyl, C₂₋₄alkenyl, OH, C₁₋₄alkoxy, CO₂H, CO₂C₁₋₄alkyl and 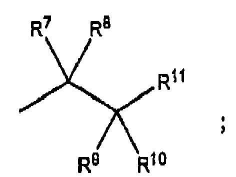 R⁵ is selected from C₁₋₄alkyl, OH, C₁₋₄alkoxy, halo, CO₂H, CO₂C₁₋₄alkyl, NH₂ and NHR¹²; R⁶ is selected from H, C₁₋₄alkyl, OH and C₁₋₄alkoxy;
R⁷, R⁸, R⁹ and R¹⁰ are each independently H and C₁₋₄alkyl or R⁷ and R⁸ together form an oxo group or R⁷ and R⁹ form a bond;
R¹¹ is selected from CH(CO₂H)NH₂, CH(CO₂C₁₋₄aklyl)NH₂, C(O)CO₂H, C(O)CO₂C₁₋₄alkyl, C(O)H, CO₂H, CO₂C₁₋₄alkyl, C(O)NH₂, C(O)NHR¹³, CH₂NH₂, CH₂NHC₁₋₄alkyl and CH₂N(C₁₋₄alkyl)₂;
R¹² is selected from H, C₁₋₄alkyl and C(O)H; and
R¹³ is H, C₁₋₄alkyl and optionally substituted phenyl, wherein optionally substituted phenyl is optionally substituted with one or more, C₁₋₄alkyl, OH, C₁₋₄alkoxy, CO₂H, CO₂C₁₋₄alkyl, halo, NH₂, NHC₁₋₄alkyl and N(C₁₋₄alkyl)₂.

Even more preferably, said IDO-mediated tryptophan metabolite is 3-HKA, 3HAA, PA or QA.

In another preferred embodiment, said IDO mediated tryptophan metabolite derivative is a compound of formula (II): 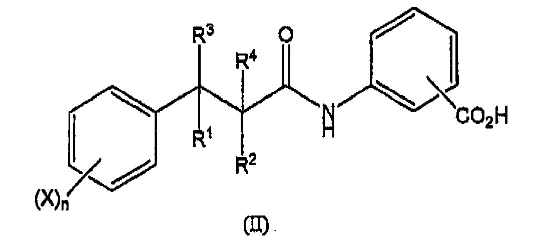 wherein each of R¹ and R² is independently selected from a hydrogen atom or a C₁-C₄alkyl group, R³ and R⁴ are each hydrogen atoms or together form another chemical bond, each X is independently selected from a hydroxyl group, a halogen atom, a C₁-C₄alkyl group or a C₁-C₄alkoxy group, or when two X groups are alkyl or alkoxy groups, they may be connected together to form a ring, and n is an integer from 1 to 3.

It should be understood that the T_{H}1 cell which is the subject of modulation in accordance with the method of the present invention is localised in a mammal, therefore requiring the subject method to be performed *in vivo.* Where the subject cell is one of a group of cells or a tissue, either isolated or not, the subject method may modulate the functioning of all the T_{H}1 cells in that group or just a subgroup of T_{H}1 cells in that group. Similarly, in the context of the modulation of the biological functioning of a mammal, it should be understood that the subject modulation may be achieved in the context of modulating T_{H}1 cell functioning either systematically or in a localised manner. Still further, irrespective of which means is employed, the cellular impact of the change in T_{H}1 cell functioning may occur in the context of either all cells or just a subgroup of cells within the relevant environment.

Reference to "down-regulating" the functional activity of a T_{H}1 cell should be understood as a reference to preventing, reducing (eg. slowing) or otherwise inhibiting one or more aspects of said activity while reference to "up-regulating" in this context should be understood to have the converse meaning.

The term "mammal" as used herein includes humans, primates, livestock animals (eg. sheep, pigs, cattle, horses, donkeys), laboratory test animals (eg. mice, rabbits, rats, guinea pigs), companion animals (eg. dogs, cats) and captive wild animals (eg. foxes, kangaroos, deer). Preferably, the mammal is human or a laboratory test animal. Even more preferably, the mammal is a human.

Although the preferred method is to downregulate T_{H}1 cell functioning, it may also be desired to induce the upregulation of this activity in certain circumstances. For example, in certain conditions the administration of a metabolite or compound of formula (I), as hereinbefore defined, may be an appropriate systemic therapy. Accordingly, a side effect of such therapy may well be unwanted downregulation of T_{H}1 cell functioning in certain cell groups or at certain tissue sites. To the extent that it is not possible to rectify this situation by ceasing administration of the metabolite or compound of formula (I), it may be desirable to administer, (in a site directed manner, for example) an antagonistic agent of that molecule. In another example, therapy with a metabolite or compounds of formula (I) may necessitate the use of antagonists of these molecules in order to inhibit the functioning of the compound which has been introduced to a mammal but which functional activity is required to be slowed or stopped. Reference to "inhibited T_{H}1 cell functioning" should therefore be understood to mean that at least some of the T_{H}1 cell functioning of the mammal exhibits inhibited, slowed or otherwise retarded functioning due to the effects of the subject metabolite or compound of formula (I) or formula (II) or a pharmaceutically acceptable salt thereof.

Accordingly, another aspect of the present invention is directed to a method of upregulating, in a mammal, inhibited T_{H}1 cell functioning, said method comprising administering to said mammal an effective amount of an antagonist of an IDO-mediated tryptophan metabolite or compound of formula (I) or formula (II) or a pharmaceutically acceptable salt thereof.

Reference to "antagonist" should be understood as a reference to any proteinaceous or non-proteinaceous molecule which directly or indirectly inhibits, retards or otherwise downregulates the cell functioning inhibitory activity of the metabolite or compounds of formula (I) or formula (II) or pharmaceutically acceptable salts thereof. Identification of antagonists suitable for use in the present invention can be routinely achieved utilising methods well known to those skilled in the art.

A further aspect of the present invention relates to the use of the invention in relation to the treatment and/or prophylaxis of disease conditions or other unwanted conditions or a predisposition to the onset of such a condition. More particularly, the present invention is directed to the treatment of disease conditions characterised by aberrant or unwanted T_{H}1 cell functioning, such as autoimmune T_{H}1 responsiveness. Without limiting the present invention to any one theory or mode of action, conditions which may be treated in accordance with the method of the present invention include, but are not limited to T_{H}1-mediated autoimmune conditions. Preferably, said condition is an autoimmune demyelinating disease of the central nervous system or periphery, such as multiple sclerosis, acute inflammatory polyradiculopathy (Guillan-Barre syndrome), polyradiculoneuropathy or chronic inflammatory demyelination.

Accordingly, another aspect of the present invention is directed to a method for the treatment and/or prophylaxis of a condition characterised by aberrant T_{H}1 cell functioning in a mammal, said method comprising administering to said mammal an effective amount of one or more IDO-mediated tryptophan metabolites or derivatives thereof for a time and under conditions sufficient to down-regulate said T_{H}1 functioning.

More particularly, there is provided a method for the treatment and/or prophylaxis of a condition characterised by autoimmune T_{H}1 cell functioning in a mammal, said method comprising administering to said mammal an effective amount of one or more IDO-mediated tryptophan metabolites or derivatives thereof for a time and under conditions sufficient to skew a T_{H}1 cell response to a T_{H}2 cell response.

Preferably, said metabolite or derivative thereof up-regulates T_{H}2 cytokine production.

More preferably, said autoimmune T_{H}1 cell is directed to a myelin protein. Still more preferably, said myelin protein is myelin basic protein.

According to this preferred aspect of the present invention, there is provided a method for the treatment and/or prophylaxis of a condition characterised by autoimmune T_{H}1 cell functioning in a mammal, which autoimmune T_{H}1 cell is directed to myelin basic protein, said method comprising administering to said mammal an effective amount of one or more IDO-mediated tryptophan metabolites or derivatives thereof for a time and under conditions sufficient to skew a T_{H}1 cell response to a T_{H}2 cell response wherein said metabolite or derivative thereof up-regulates T_{H}2 cytokine production.

Preferably, said condition is an autoimmune demyelinating disease of the central nervous system or periphery. More preferably, said peripheral demyelinating disease is acute inflammatory polyradiculopathy, polyradiculoneuropathy or chronic inflammatory demyelination.

Most preferably, said condition is multiple sclerosis.

In a preferred embodiment, the IDO-mediated tryptophan metabolite is a compound of formula (I): 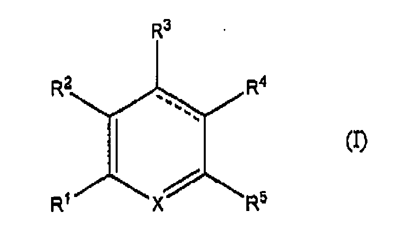 wherein
X is selected from N and CR⁶;
- ---- represents a single or double bond;
   R¹ is selected from H, C₁₋₄alkyl, OH, C₁₋₄alkoxy, halo, CO₂H and CO₂C₁₋₄alkyl;
   R² is selected from H, C₁₋₄alkyl, OH, C₁₋₄alkoxy, halo, or R¹ and R² together form an optionally substituted fused phenyl ring;
   R³ is selected from H, C₁₋₄alkyl, OH, C₁₋₄alkoxy and halo;
   R⁴ is selected from H, C₁₋₄alkyl, C₂₋₄alkenyl, OH, C₁₋₄alkoxy, CO₂H, CO₂C₁₋₄alkyl and 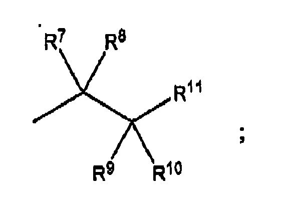 R⁵ is selected from C₁₋₄alkyl, OH, C₁₋₄alkoxy, halo, CO₂H, CO₂C₁₋₄alkyl, NH₂ and NHR¹²; R⁶ is selected from H, C₁₋₄alkyl, OH and C₁₋₄alkoxy;
   R⁷, R⁸, R⁹ and R¹⁰ are each independently H and C₁₋₄alkyl or R⁷ and R⁸ together form an oxo group or R⁷ and R⁹ form a bond;
   R¹¹ is selected from CH(CO₂H)NH₂, CH(CO₂C₁₋₄alkyl)NH₂, C(O)CO₂H, C(O)CO₂C₁₋₄alkyl, C(O)H, CO₂H, CO₂C₁₋₄alkyl, C(O)NH₂, C(O)NHR¹³, CH₂NH₂, CH₂NHC₁₋₄alkyl and CH₂N(C₁₋₄alkyl)₂;
   R¹² is selected from H, C₁₋₄alkyl and C(O)H; and
   R¹³ is H, C₁₋₄alkyl and optionally substituted phenyl, wherein optionally substituted phenyl is optionally substituted with one or more, C₁₋₄alkyl, OH, C₁₋₄alkoxy, CO₂H, CO₂C₁₋₄alkyl, halo, NH₂, NHC₁₋₄alkyl and N(C₁₋₄alkyl)₂.

Preferably, said IDO-mediated tryptophan metabolite is 3-HKA, 3-HAA, PA or QA. In another preferred embodiment, said IDO-mediated tryptophan metabolite derivative is a compound of formula (II); 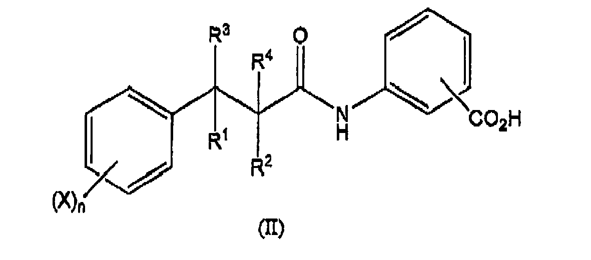 wherein each of R¹ and R² is independently selected from a hydrogen atom or a C₁-C₄alkyl group, R³ and R⁴ are each hydrogen atoms or together form another chemical bond, each X is independently selected from a hydroxyl group, a halogen atom, a C₁-C₄alkyl group or a C₁-C₄alkoxy group, or when two X groups are alkyl or alkoxy groups, they may be connected together to form a ring, and n is an integer from 1 to 3.

The metabolites, derivatives and compounds of formula (I), formula (II) or pharmaceutically acceptable salts thereof may also be used in conjunction with another therapy, for example an immunosuppressive or anti-inflammatory treatment regime to the extent that an autoimmune condition is being treated.

An "effective" amount means an amount necessary at least partly to attain the desired response, or to delay the onset or inhibit progression or halt altogether, the onset or progression of a particular condition being treated. The amount varies depending upon the health and physical condition of the individual to be treated, the taxonomic group of individual to be treated, the degree of protection desired, the formulation of the composition, the assessment of the medical situation, and other relevant factors. It is expected that the amount will fall in a relatively broad range that can be determined through routine trials.

Reference herein to "treatment" and "prophylaxis" is to be considered in its broadest context. The term "treatment" does not necessarily imply that a subject is treated until total recovery. Similarly, "prophylaxis" does not necessarily mean that the subject will not eventually contract a disease condition. Accordingly, treatment and prophylaxis include amelioration of the symptoms of a particular condition or preventing or otherwise reducing the risk of developing a particular condition. In the context of multiple sclerosis, for example, this may include the amelioration or prevention of inflammation of some neural regions but not necessarily all neural regions. This could occur, for example, where the subject compound is administered locally into some but not all affected tissue. The term "prophylaxis" may be considered as reducing the severity or onset of a particular condition. "Treatment" may also reduce the severity of an existing condition.

Administration of the compounds of formula (I), formula (II) or pharmaceutically acceptable salts thereof or antagonist thereof (herein referred to as "modulatory agent"), in the form of a pharmaceutical composition, may be performed by any convenient means. The modulatory agent of the pharmaceutical composition is contemplated to exhibit therapeutic activity when administered in an amount which depends on the particular case. The variation depends, for example, on the human or animal and the modulatory agent chosen. A broad range of doses may be applicable. Considering a patient, for example, from about 0.1 mg to about 1 mg of modulatory agent may be administered per kilogram of body weight per day. Dosage regimes may be adjusted to provide the optimum therapeutic response. For example, several divided doses may be administered daily, weekly, monthly or other suitable time intervals or the dose may be proportionally reduced as indicated by the exigencies of the situation.

The modulatory agent may be administered in a convenient manner such as by the oral, intravenous (where water soluble), intraperitoneal, intramuscular, subcutaneous, intradermal or suppository routes or implanting (eg. using slow release molecules). The modulatory agent may be administered in the form of pharmaceutically acceptable nontoxic salts, such as acid addition salts or metal complexes, eg. with zinc, iron or the like (which are considered as salts for purposes of this application). Illustrative of such acid addition salts are hydrochloride, hydrobromide, sulphate, phosphate, maleate, acetate, citrate, benzoate, succinate, maleate, ascorbate, tartrate and the like. If the active ingredient is to be administered in tablet form, the tablet may contain a binder such as tragacanth, corn starch or gelatin; a disintegrating agent, such as alginic acid; and a lubricant, such as magnesium stearate.

The modulatory agent may be linked, bound or otherwise associated with any proteinaceous or non-proteinaceous molecules. For example, in one embodiment of the present invention said modulatory agent may be associated with a molecule which permits targeting to a localised region.

Routes of administration include, but are not limited to, respiratorally, intratracheally, nasopharyngeally, intravenously, intraperitoneally, subcutaneously, intracranially, intradermally, intramuscularly, intraoccularly, intrathecally, intracereberally, intranasally, infusion, orally, rectally, *via* IV drip, patch and implant.

In accordance with these methods, the agent defined in accordance with the present invention may be coadministered with one or more other compounds or molecules. By "coadministered" is meant simultaneous administration in the same formulation or in two different formulations via the same or different routes or sequential administration by the same or different routes. For example, the subject agent may be administered together with an agonistic agent in order to enhance its effects. By "sequential" administration is meant a time difference of from seconds, minutes, hours or days between the administration of the two types of molecules. These molecules may be administered in any order.

Yet another aspect of the present invention is directed to the use an IDO-mediated tryptophan metabolite or derivative thereof in the manufacture of a medicament for the treatment of a condition characterised by aberrant T_{H}1 cell functioning wherein administering said compound down-regulates said T_{H}1 cell functioning.

Preferably, said condition is an autoimmune condition and even more preferably an autoimmune condition characterised by an immune response directed to a myelin protein. More preferably, said condition is an autoimmune demyelinating disease of the CNS or periphery. Most preferably, said condition is multiple sclerosis.

Yet another aspect of the present invention is directed to the use of an IDO-mediated tryptophan metabolite or derivative thereof in the manufacture of a medicament for the treatment of multiple sclerosis.

In one embodiment, said IDO-mediated tryptophan metabolite is a compound of formula (I): 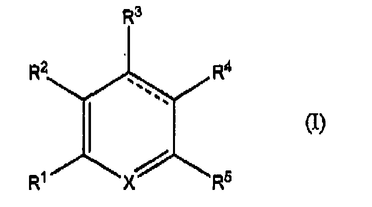 wherein
X is selected from N and CR⁶;
----- represents a single or double bond;
R¹ is selected from H, C₁₋₄alkyl, OH, C₁₋₄alkoxy, halo, CO₂H and CO₂C₁₋₄alkyl;
R² is selected from H, C₁₋₄alkyl, OH, C₁₋₄alkoxy, halo, or R¹ and R² together form an optionally substituted fused phenyl ring;
R³ is selected from H, C₁₋₄alkyl, OH, C₁₋₄alkoxy and halo;
R⁴ is selected from H, C₁₋₄alkyl, C₂₋₄alkenyl, OH, C₁₋₄alkoxy, CO₂H, CO₂C₁₋₄alkyl and 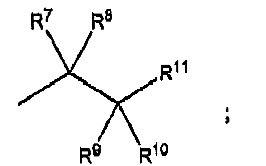 R⁵ is selected from C₁₋₄alkyl, OH, C₁₋₄alkoxy, halo, CO₂H, CO₂C₁₋₄alkyl, NH₂ and NHR¹²; R⁶ is selected from H, C₁₋₄alkyl, OH and C₁₋₄alkoxy;
R⁷, R⁸, R⁹ and R¹⁰ are each independently H and C₁₋₄alkyl or R⁷ and R⁸ together form an oxo group or R⁷ and R⁹ form a bond;
R¹¹ is selected from CH(CO₂H)NH₂, CH(CO₂C₁₋₄alkyl)NH₂, C(O)CO₂H, C(O)CO₂C₁₋₄alkyl, C(O)H, CO₂H, CO₂C₁₋₄alkyl, C(O)NH₂, C(O)NHR¹³, CH₂NH_{2;} CH₂NHC₁₋₄alkyl and CH₂N(C₁₋₄alkyl)₂;
R¹² is selected from H, C₁₋₄alkyl and C(O)H; and
R¹³ is H, C₁₋₄alkyl and optionally substituted phenyl, wherein optionally substituted phenyl is optionally substituted with one or more, C₁₋₄akyl, OH, C₁₄alkoxy, CO₂H, CO₂C₁₋₄alkyl, halo, NH₂, NHC₁₋₄alkyl and N(C₁₋₄alkyl)₂.

Preferably, said IDO-mediated tryptophan metabolite is 3-HKA, 3HAA, PA or QA.

In another preferred embodiment, said IDO mediated tryptophan metabolite derivative is a compound of formula (II): 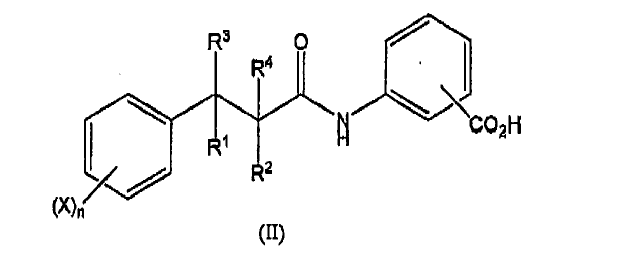 wherein each of R¹ and R² is independently selected from a hydrogen atom or a C₁-C₄alkyl group, R³ and R⁴ are each hydrogen atoms or together form another chemical bond, each X is independently selected from a hydroxyl group, a halogen atom, a C₁-C₄alkyl group or a C₁-C₄alkoxy group, or when two X groups are alkyl or alkoxy groups, they may be connected together to form a ring, and n is an integer from 1 to 3.

The present invention contemplates the administration of the subject metabolites either alone or as a pharmaceutical composition comprising said metabolite or a pharmaceutically acceptable salt thereof or antagonist thereof as hereinbefore defined and one or more pharmaceutically acceptable carriers and/or diluents. Said agents are referred to as the active ingredients.

The pharmaceutical forms suitable for injectable use include sterile aqueous solutions (where water soluble) or dispersions and sterile powders for the extemporaneous preparation of sterile injectable solutions or dispersion or may be in the form of a cream or other form suitable for topical application. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol and liquid polyethylene glycol, and the like), suitable mixtures thereof, and vegetable oils. The proper fluidity can be maintained, for example, by the use of a coating such as lecithin, by the maintenance of the required particle size in the case of dispersion and by the use of superfactants. The preventions of the action of microorganisms can be brought about by various antibacterial and antifungal agents, for example, parabens, chlorobutanol, phenol, sorbic acid, thimerosal and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various of the other ingredients enumerated above, as required, followed by filtered sterilisation. Generally, dispersions are prepared by incorporating the various sterilised active ingredient into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

When the active ingredients are suitably protected they may be orally administered, for example, with an inert diluent or with an assimilable edible carrier, or it may be enclosed in hard or soft shell gelatin capsule, or it may be compressed into tablets, or it may be incorporated directly with the food of the diet. For oral therapeutic administration, the active compound may be incorporated with excipients and used in the form of ingestible tablets, buccal tablets, troches, capsules, elixirs, suspensions, syrups, wafers, and the like. Such compositions and preparations should contain at least 1% by weight of active compound. The percentage of the compositions and preparations may, of course, be varied and may conveniently be between about 5 to about 80% of the weight of the unit. The amount of active compound in such therapeutically useful compositions in such that a suitable dosage will be obtained. Preferred compositions or preparations according to the present invention are prepared so that an oral dosage unit form contains between about 0.1 µg and 2000 mg of active compound.

The tablets, troches, pills, capsules and the like may also contain the components as listed hereafter: a binder such as gum, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a favouring agent such as peppermint, oil of wintergreen, or cherry flavouring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavouring such as cherry or orange flavour. Of course, any material used in preparing any dosage unit form should be pharmaceutically pure and substantially non-toxic in the amounts employed. In addition, the active compound(s) may be incorporated into sustained-release preparations and formulations.

Yet another aspect of the present invention relates to the metabolites or derivatives as hereinbefore defined or pharmaceutically acceptable salts thereof or antagonists thereof, as hereinbefore defined, when used in the method of the present invention.

It should also be understood that the present invention extends to methods of up-regulating T_{H}2 functioning based on skewing a T_{H} cell response towards this subclass. This is achieved in accordance with the methods discussed herein wherein the application of the disclosed methodology as it is directed to skewing a T_{H}1 response will inherently achieve both the down-regulation of a T_{H}1 response and the simultaneous up-regulation of a T_{H}2 response. Since the T_{H}2 response is supportive of the humoral response, up-regulation of the T_{H}2 response permits the rational design of therapeutic and/or prophylactic regimes which benefit from the induction of such an immune response outcome.

The invention also provides a pharmaceutical pack or kit comprising one or more containers filled with one or more of the ingredients of the pharmaceutical compositions of the invention. Associated with such container(s) can be a notice in the form prescribed by a governmental agency regulating the manufacture, use or sale of pharmaceuticals or biological products, which notice reflects approval by the agency of manufacture, use or sale for human administration.

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

All publications and patent applications cited in this specification are herein incorporated by reference as if each individual publication or patent application were specifically and individually indicated to be incorporated by reference.

The present invention has been described in terms of particular embodiments found or proposed by the present inventor to comprise preferred modes for the practice of the invention. It will be appreciated by those of skill in the art that, in light of the present disclosure, numerous modifications and changes can be made in the particular embodiments exemplified without departing from the intended scope of the invention. For example, due to codon redundancy, changes can be made in the underlying DNA sequence without affecting the protein sequence. Moreover, due to biological functional equivalency considerations, changes can be made in protein structure without affecting the biological action in kind or amount. All such modifications are intended to be included within the scope of the appended claims. The reference to any prior art in this specification is not, and should not be taken as, an acknowledgment or any form of suggestion that that prior art forms part of the common general knowledge in Australia. The present invention is further defined by the following non-limiting examples:

### EXAMPLE 1

### TREATMENT OF ESTABLISHED AUTOIMMUNE NEUROINFLAMMATION WITH 3,4-DAA, AN ORALLY ACTIVE SYNTHETIC TRP METABOLITE

### Results

Gene transcripts that are differentially regulated in T cells treated with APL were identified. Thus, a T cell line was generated from mice with a transgenic TCR specific for the myelin basic protein (MBP) peptide Ac1-11 and a gene chip analysis performed. The altered peptide ligand Ac1-11[4Y] binds to the major histocompatibility complex (MHC) class II I-A" with greater affinity than the native peptide. Whereas Ac1-11 induces primarily a T_{H}1 response, Ac1-11[4Y] promotes a T_{H}2 response (Pearson et al., J Exp Med. 185:583-99, 1997). Microarray analyses revealed that transcripts for mdoleamine-2,3-dioxygenase (IDO) were over 70-fold upregulated after 48h in Ac1-11[4Y]-activated T cells compared to Ac1-11-activated cells (Table 1).

| **Table 1: T cell transcripts specifically induced by APL MBP Ac1-11 [4Y]** | | |
|---|---|---|
| **Accession** | **Description** | **Fold** **Change** |
| **X12531** | Mouse mRNA for macrophage inflammatory protein (MIP). | 164.8 |
| **M69109** | Mouse indoleamine 2,3-dioxygenase mRNA, complete cds. | 74.4 |
| **aa028770** | mi15h02.r1 Scares mouse p3NMF19.5 Mus musculus cDNA clone 463635 5', mRNA sequence. | 34.1 |
| **M10937** | Mouse epidermal 67-kDa type II keratin mRNA. | 21 |
| **X03532** | Mouse mRNA for IgG1 induction factor. | 18.2 |
| **u29947** | Mus musculus alpha-D-mamiosidase (Man2b1) mRNA, complete cds. | 14.7 |
| **aa572259** | v152b09.r1 Stratagene mouse skin (#937313) Mus musculus cDNA clone 975833 5', mRNA sequence. | 14.4 |
| **M12279** | Mouse interferon-induced Mx protein mRNA conferring selective resistance to influenza virus, complete cds. | 11.8 |
| **x03019** | Mouse mRNA for granulocyte-macrophage colony stimulating factor (GM-CSF). | 10.8 |
| **x51834** | Murine gene for osteopontin. | 10.6 |
| **U96700** | Mus musculus serine proteinase inhibitor 6 (SPI6) mRNA, complete cds. | 10.6 |
| **Msa.1376.0** | Mouse mRNA for early T-lymphoeyte activation 1 protein (ETa-1) | 10.4 |
| **M35590** | Mouse macrophage inflammatory protein 1-beta (MIP-1) mRNA, complete cds. | 9.8 |
| **Msa.34983.0** | Homologous to sp P10923; OSTEOPONTIN PRECURSOR (BONE SIALOPROTEIN 1) (MINOPONTIN) (EARLY T LYMPHOCYTE ACTIVATION 1 PROTEIN) (SECRETED PHOSPHOPROTEIN 1) (SPP-1) (2AR) (CALCIUM OXALATE CRYSTAL GROWTH INHIBITOR PROTEIN). | 9.7 |
| **Msa.757.0** | Mouse chromatin nonhistone high mobility group protein (HGM-1(Y), complete cds | 8.9 |
| **d89571** | Mus musculus mRNA for ryudocan core protein, complete cds. | 8.8 |
| **Maa.27449.0** | Homologous to sp P10923; OSTEOPONTIN PRECURSOR (BONE SIALOPROTEIN 1) (MINOPONTIN) (EARLY T LYMPHOCYTE ACTIVATION 1 PROTEIN) (SECRETED PHOSPHOPROTEIN 1) (SPP-1) (2AR) (CALCIUM OXALATE CRYSTAL GROWTH INHIBITOR PROTEIN). | 8.7 |
| **x03019** | Mouse mRNA for granulocyte-macrophage colony stimulating factor (GM-CSF). | 8.4 |
| **x06271** | Murine gene for interleukin 5 (eosinophil differentiation factor). | 8.1 |
| **Msa.17355.0** | Homologous to sp P48960: LEUCOCYTE ANTIGEN CD97 PRECURSOR | 7.9 |
| **u85786** | Mus musculus sodium channel beta-1 subunit mRNA, complete cds. | 7.6 |
| **x14045** | Mouse mRNA for T-cell growth factor P40. | 7.2 |
| **x02732** | Mouse gene for interleukin-3 (Il-3). | 6.9 |
| **ET61010** | Mouse lymphocyte mRNA for T-cell receptor beta, partial cds. | 6.5 |
| **aa185666** | mt89d08.r1 Soares mouse lymph node NbMLN Mus musculus cDNA clone 637071 5', mRNA sequence. | 6.4 |
| **Msa.2173.0** | M.musculus mRNA for inhibin beta-A subunit | 6.1 |
| **u81603** | Mus musculus Bya2 homolog (Eya2) mRNA, complete cds. | 5.8 |
| **Msa.1189.0** | Mus musculus secreted T cell protein (P500/TCA3; SIS-epsilon) mRNA, complete cds | 5.8 |
| **aa709719** | **vt38a10.r1** Barstead mouse proximal colon MPLRB6 Mus musculus cDNA clone 1165338 5', mRNA sequence. | 5.6 |
| **Msa.3279.0** | Mus musculus ROS-r protein mRNA, complete ods | 5.6 |
| **Msa.2937.0** | M.musculus mRNA for mTGIF protein | 5.6 |
| **U06119** | Mus musculus cathepsin H prepropeptide (ctsH) mRNA, complete cds. | 5.4 |
| **u08372** | Mus musculus Balb/c asialoglycoprotein receptor (MHL-1) mRNA, complete cds. | 5.3 |
| **AA389997** | vb43h11.r1 Soares mouse lymph node NbMLN Mus musculus cDNA clone 751749 5', mRNA sequence. | 5.3 |
| **aa163485** | mt66b03.r1 Soares mouse lymph node NbMLN Mus musculus cDNA clone 634829 5', mRNA sequence. | 5 |

Tarp metabolites generated by IDO include 3-Hydroxykynurenic acid (3-HKA), 3-Hydroxyanthranilic acid (3-HAA), picolinic acid (PA) and quinolinic acid (QA)
(R. Schwarez, Curr. Opin. Pharmacol. 4:12-7, 2004). To test the hypothesis that that kynurenines play a role in the activation of myelin-specific T cells, splenocytes isolated from B10.PL mice with a transgenic T cell receptor (TCR) specific for the myelin peptide myelin basic protein (MBP) peptide Ac1-11 were stimulated with MBP Ac1-11 in combination with the Trp metabolites PA, QA, 3-HAA, 3-HKA and the synthetic derivative 3,4-DAA. 3,4-DAA shares the anthranilic acid core with 3-HKA and 3-HAA (Fig. 1A) and is an orally active compound with favorable pharmacokinetics in humans (Isaji et al., Cardiovascular Drug Reviews, 16:288-299, 1998). There was dose-dependent suppression of antigen-specific proliferation of MBPAc1-11 TCR transgenic CD4+ cells by 3-HAA, 3-HKA and 3,4-DAA (Fig. 1B). Suppression of T cell response by 3,4-DAA was not due to cytotoxicity but instead was associated with a G1/S phase arrest in CD4 cells. T_{H}1-mediated autoimmune diseases, such as experimental autoimmune encephalomyelitis (EAE), an animal model of multiple sclerosis, are caused by auto-reactive CD4⁺ cells secreting pro-inflammatory cytokines such as IFN-γ and TNF-α (L. Steinman, J Exp Med. 197:1065-71, 2003). Thus, the effect of Trp metabolites on the cytokine profile of MBPAc1-11 stimulated TCR transgenic splenocytes was analysed. Both natural Trp metabolites and 3,4-DAA reduced release of IL-2 and the T_{H}1 cytokines IFN-γ and TNF-α from activated CD4 cells. Conversely, the T_{H}2 cytokines IL-4 and IL-10 were up-regulated in MBPAc1-11 TCR transgenic T cells after stimulation with antigen (Fig. 1C, Table 3). Thus, both natural Trp metabolites and 3,4-DAA skew the cytokine profile of myelin-specific T helper cells from a T_{H}1 to T_{H}2 phenotype.

**Table 3: Modulation of splenocyte cytokine profile by Trp metabolites**

| | *IL-2* | *IFN-*γ | *TNF-*α | *IL-4* | *IL-10* | *IL-6* | *IL-12*/*23* |
|---|---|---|---|---|---|---|---|
| Control | **212 ±20** | **2159 ±250** | **218 ±13** | **31 ±3** | **74 ±1** | **364 ±10** | **199 ±4** |
| PA | **256 ±12** | **1114 ±50*** | **150 ±4*** | **54 ±3*** | **138 ±11*** | **363 ±12** | **141 ±16*** |
| QA | **233 ±25** | **1237 ±24*** | **191 ±9** | **56 ±2*** | **84 ±2** | **367 ±8** | **116 ±2**** |
| 3-HKA | **212 ±2** | **2031 ±286** | **141 ±6*** | **39 ±3** | **122 ±12*** | **251 ±10*** | **133 ±2*** |
| 3-HAA | **144 ±9*** | **184 ±38***** | **116 ±12*** | **37 ±3** | **134 ±23*** | **205 ±16*** | **85 ±5**** |
| 3,4-DAA | **66 ±10**** | **115 ±32***** | 5**0 ±1**** | **82 ±2**** | **500 ±46***** | **317 ±11** | **96 ±12*** |

| | | | | | | | |
|---|---|---|---|---|---|---|---|
| Splenocytes from MBPAc1-11 TCR transgenic mice were activated with MBPAc1-11 (0.5 - 2.5µg/ml) in the absence or presence of Trp metabolites PA, QA, 3-HKA, 3-HAA, 3,4-DAA at 200 µM (IL-2, IFN-γ, TNF-α, IL-6 and IL-12/23 p40) or 30 µM (IL-4, IL-10). Cytokine release was measured after 48h (IL-2, IL-6, IL-12/23 p40), 72h (IFN-γ, TNF-α) or 120h (IL-4, IL-10) using ELISA. | | | | | | | |

The effects of 3,4-DAA on myelin-specific T cells *in vivo* were examined. MBPAc1-11 TCR transgenic mice were fed with 3,4-DAA for 5 days. When splenocytes were stimulated with MBPAc1-11 *ex vivo* there was a suppression of MBPAc1-11-specific T cell proliferation. Similarly, antigen-induced release of the pro-inflammatory cytokines IFN-γ, TNF-α and IL-12/23 p40 was profoundly suppressed in splenocytes from 3,4-DAA-treated mice (Fig. 2A), indicating that 3,4-DAA is orally active to suppress the generation of antigen-specific autoreactive T_{H}1 cells. Moreover, FACS analysis revealed a downregulation of the expression of MHC class II and costimulatory molecules on CD11b+ monocytes (Fig. 2B, Table 3), indicating that 3,4-DAA suppresses the activation of antigen-presenting cells *in vivo*. Efficient presentation of antigens to CD4⁺ T_{H} cells requires presentation of the antigen on MHC class II molecules and delivery of costimulatory molecules such as CD40, CD80, and CD86. The expression of MHC class II, and costimulatory molecules is induced by IFN-γ (Carreno et al., Annu Rev Immunol. 20:29-53, 2002).

To assess the effect of Trp metabolites on antigen-presentation, EOC20 microglial cells were used as a model. EOC20 cells express MHC class II and costimulatory molecules constitutively at low levels which rapidly upregulate by IFN-γ. 3,4-DAA induced a dose-dependent decrease of MHCII and costimulatory molecule expression induced by IFN-γ, while constitutive expression of these molecules remained unchanged (Fig. 2C). Of note, 3,4-DAA did not affect cell viability at concentrations up to 200 µM as assessed by propidium iodine staining, 3,4-DAA-mediated suppression of EFN-γ-induced MHC class II expression in EOC20 calls was paralleled by an inhibition of the class II transactivator CIITA (Fig. 2D). In addition, 3,4-DAA suppressed expression of inducible nitric oxide synthase (iNOS) and nitric oxide (NO) release from EOC20 cells induced by IFN-γ and lipopolysaccharide (LPS) (Fig. 2E, F). Thus, 3,4-DAA interferes with IFN-γ signalling in general. When EOC20 cells were preincubated with 3,4-DAA, phosphorylation of STAT1α induced by IFN-γ was dose-dependently inhibited (Fig. 2G). Thus, 3,4-DAA inhibits the activation of antigen-presenting cells by interfering with IFN-γ signalling.

To assess whether 3,4-DAA suppresses the function of autoreactive T_{H}1, the compound was tested in an autoimmune disease model. Immunization of SJ/L mice with PLP₁₃₉₋₁₅₁ induces relapsing-remitting EAE, a prototypical animal model of multiple sclerosis (L. Steinman, Nat Immunol. 2:762-4, 2001); Patients with relapsing-remitting multiple sclerosis are typically treated after the first on set of clinical symptoms to prevent further attacks. Thus, to resemble the clinical setting, treatment was initiated in the animals after the onset of disease when the animals reached their peak functional incapacitation. After randomization according to clinical score, mice were treated twice daily starting at day 15 or 16 after immunization by oral gavage. The majority of animals recovered after the initial acute phase. While vehicle-treated animals displayed severe relapses throughout the course of disease, animals treated with 3,4-DAA showed little clinical signs of a relapsing disease (Fig. 3A). At several dose levels there was significant reduction in clinical disease index (CDI) and peak relapse score (Table 2).

**Table 2: 3,4-DAA ameliorates clinical symptoms of established EAE**

| *Treatment [mglkg*/*d]* | *Onset [dpt]* | *Peak Score* *[acute]* | *CDI* | *CDI [d0-26]* | *CDI[d27-60]* | *Peak Score* *[relapse]* |
|---|---|---|---|---|---|---|
| | | | | | | |
| vehicle | **12.7**+/-**0.3** | **3.0**+/-**0** | **109**+/-**12** | **27.3**+/-**3.2** | **81**+/-**10** | **3.0**+/-**0.4** |
| 30 | **13.3**+/-**0.2** | **3.0**+/-**0** | **76**+/-**13** | **32.3**+/-**1.9** | **54**+/-**13** | **2.9**+/-**0.4** |
| 100 | **12.7**+/-**0.2** | **3.1**+/-**0.3** | **51**+/-**11***** | **23.3**+/-**3.4** | **28**+/-**9***** | **1.4**+/-**0.3**** |
| 200 | **14.1**+/-**0.8** | **194**+/-**0.4** | **45**+/-**16**** | **17.2**+/-**5.9** | **M**+/-**10***** | **2.0**+/-**0.3**** |
| 300 | **12.9**+/-**0.3** | **3.1**+/-**0.3** | **61**+/-**12*** | **23.9**+/-**3.1** | **37**+/-**9**** | **1.9**+/-**0.5** |
| 500 | **13.1**+/-**0.5** | **2.8**+/-**0.5** | **68**+/-**16** | **21.7**+/-**5.7** | **47**+/-**11** | **2.2**+/-**0.3*** |

| | | | | | | |
|---|---|---|---|---|---|---|
| 7-8 wk-old female SJ/L mice were immunized with PLP₁₃₉₋₁₅₁ and treated with vehicle alone (Na-CMC) or 3,4-DAA at the concentrations indicated. CDI (cumulative disease index) was calculated as the sum of scores over the period of 60 day or over the period indicated. *p<0.05, **p<0.01, ***p<0.001. | | | | | | |

Interestingly, a bell shaped dose-response curve was observed with the most effective dose between 100 and 200mg/kg/d (Table 2). This may be due to the unique and distinct mechanisms altered by 3,4-DAA, which may have opposing effects on EAE. First, 3,4-DAA suppresses the generation of IFN-γ by activated myelin specific T cells (Table 1). Moreover, 3,4-DAA abrogates IFN-γ-aignalling in antigen presenting cells (Fig. 3). Suppression of IFN-γ-aignalling through genetic ablation of TFN-γR or neutralizing antibodies has been shown to worsen EAE. Moreover, genetic ablation of STAT1 leads to multiple organ inflammation (Wang et al., Proc Natl Acad Sci USA. 99:16209-14, 2002) probably due to impaired development of T_{R} cells (Nishibori et al., J Exp Med. 199:25-34, 2004) and STAT1-deficient mice develop more severe EAE than wildtype mice (Bettelli et al., J Exp Med. 200:79-87, 2004). In addition NOD mice may be predisposed to autoimmunity due to a defect in the signalling of IFN-γ through STAT1, which leads to impaired induction of Trp catabolism (Grohmann et al., J Exp Med. 198:153-60, 2003). On the other hand, 3,4-DAA suppresses the release of nitric oxide and the expression of MHCII and costimulatory molecules on antigen presenting cells (Figure 2E). It is thought that 3,4-DAA in part bypasses the requirement of Trp catabolism to suppress the generation of autoreactive T_{H}1 cells through direct suppression of myelin-specific CD4⁺ T-cells. The high micromolar doses of 3,4-DAA used in the *in vitro* assays are well achieved in vivo. Using gas chromatography steady-state plasma levels in SJ/L mice of 44.3 µM at 100 mg/kg/d and 314.9 µM at 300 mg/kg/d were observed (data not shown). Moreover, peak plasma levels after oral intake are 125 µM at 200 mg in humans (Charng et al., JFood Drug Anal. 10:135-8, 2002) and steady state plasma levels after oral intake were reported to be 52 µM at 550 mg/kg/day in mice and 50-200 µM at 600 mg/day in humans (Izawa et al., Arterioscler Thromb Vasc Biol. 21:1172-8).

Consistent with the findings that 3,4-DAA suppresses the activation of myelin specific T_{H}1 cells *in vitro,* it was found that the frequency of activated T cells was decreased in EAE-induced mice with EAE treated with 3,4-DAA. There was a 40% reduction of CD4 cells co-expressing the activation markers CD25, CD44 or CD69 (Fig. 3B). Moreover proliferation of PLP-specific T cells in response was decreased in animals treated with 3,4-DAA. In addition the release of pro-inflammatory cytokines IFN-γ, TNF-α and IL-12/23 p40 was reduced in mice treated with 3,4-DAA (Fig. 3C). Of note, ConA-induced T cell proliferation was not altered in 3,4-DAA-troated mice indicating a specific effect on PLP-specific T cells (data not shown). Next, brains and spinal cords of mice with EAE were assessed for signs of inflammation. There was a reduction of parenchymal and total inflammatory foci in CNS tissue from mice treated with 3,4-DAA compared to vehicle-treated mice (Fig. 3D). To evaluate whether activation of CNS antigen presenting cells was suppressed *in vivo,* a series of immunohistochemistry and RT-PCR experiments were conducted. As shown on figure 4A, there is strong expression of MHC class II, CD40, CD80, CD86 and iNOS in parenchymal cells with microglial morphology in spinal cords of SJ/L mice with EAE treated with vehicle. In contrast, in animals that have been treated with 3,4-DAA, expression of these molecules is drastically reduced. Moreover, RT-PCR experiments show, that expression of the class II transactivator CIITA, TNF-α and IL-12/23 p40 is reduced in spinal cords of animals treated with 3,4-DAA (Fig. 4B) implicating the suppression of antigen presenting cells as a key mechanism in the immunosuppressive effects of 3,4-DAA.
3,4-DAA therefore represents a synthetic Trp metabolite with unique immunomodulatory properties including Suppression of IL-2/23 and inhibition of signalling through STAT molecules. Trp metabolites and derivatives thereof, such as 3,4-DAA, may thus represent a novel class of drugs for the treatment of TH₁-mediated autoimmune diseases.

**Table 4: 3,4-DAA suppresses the expression of MHCII and costimulatory molecules on monocytes in vivo.**

| | *MHCII* | *CD80* | *CD86* | *CD40* |
|---|---|---|---|---|
| Vehicle | **51.9** | **9.4** | **34.2** | **41.1** |
| 3,4-DAA | **28.4** | **2.7** | **21.3** | **24.9** |

| | | | | |
|---|---|---|---|---|
| MBPAc1-11 TCR transgenic mice were treated with 3,4-DAA (500mg/kg/d) or vehicle alone (Na-CMC 0.5%) twice daily for 5 days by oral gavage. Splenocytes were analyzed by flow cytometry. Values are given as percent of positive cells of the CD11b⁺ monocyte population. Data are representative of pooled splenocytes from 3 different animals in each group. | | | | |

**Table 5: Primer sequences for semiquantitative PCR**

| | **Forward sequence (5'-3')** | **SEQ ID NO.** | **Reverse sequence (5'-3')** | **SEQ ID NO.** |
|---|---|---|---|---|
| MHCII (I-A^{k/a}) | GGATGCTTCCTGAOTTTO | 1 | CTGGTTTCATAAACGCCG | 8 |
| CIITA | ACGGTCACTACACTTAAAATG | 2 | CATAACTATAATGCTACGGGGA | 9 |
| IL-12 p35 | CCAAGGTCAGCGTTCC | 3 | GTTTGGTCCCGTGTGAT | 10 |
| IL-12/23 p40 | CACGTTTATGTTGTAGAGGTG | 4 | GTCTCGCCTCCTTTGT | 11 |
| IL-23 p19 | AATGTGCCCCGTATCC | 5 | GGAGGTGTGAAGTTGCT | 12 |
| TNF-α | CCTTGTCTACTGCTAACCGACTCCT | 6 | AGTTGGTCCCCCTTCTCCA | 13 |
| INOS | GACGGCAAACATGACT | 7 | CCACTCGTACTTGGGAT | 14 |

### Materials and Methods

*Animals.* Female SJL/J mice were purchased from the Jackson Laboratory (Bar Harbor) at 5 weeks of age. MBP Ac 1-11 TCR transgenic mice, obtained from C. Janeway Jr. (Hardardottir et al., Proc Natl Acad Set USA 92:354-8, 1995) were backcrossed into the B10.PL background. All animal protocols were approved by the Division of Comparative Medicine at Stanford University and the Committee of Animal Research at the University of California San Francisco, in accordance with the National Institutes of Health guidelines.

*Reagents.* Picolinic acid, quinolinic acid, 3-hydroxy-anthranilic acid and 3-hydroxy-kynurenic acid were purchased from Sigma. Murine recombinant IFN-γ and IL-6 were obtained from Biosource. 3,4-DAA was synthesized and provided by Angiogen Pharmaceutical Pty. Ltd. Peptides MBP Ac1-11 (Ac-ASQKRPSQRHG) (SEQ ID NO:36) and PLP p139-151 (HCLOKWLGHPDKF) (SEQ ID NO:37) were synthesized on a peptide synthesizer (model 9050; MilliGen) by standard 9-fluorenylmethoxycarbonyl chemistry, and purified by high-performance liquid chromatography (HPLC), Amino acid sequences were confirmed by amino acid analysis and mass spectroscopy. The purity of each peptide was greater than 95%.

*Microglia and macrophages.* Microglial EOC 20 cells, derived from C3H/HeJ CH-2k mice using a non-viral immortalization procedure (Walker et al., J Neuroimmunol. 63:163-74, 1995), were obtained from the American Type Culture Collection (ATCC) and were grown using DMEM media supplemented with 1 mM sodium pyruvate, 10% (v/v) fetal oalf serum (FCS) and 20% (v/v) media conditioned by LADMAC mouse bone marrow cells (ATCC, CRL-2420) as a source of CSP-1, Primary microglia were isolated from 1-3-day-old 129Sv/Ev mice as described previously (Youssef et al., Nature 420:78-84, 2002). Primary microglia were 95% CD11b+ by fluorescence-activated cell sorting (FACS). Primary macrophages (peritoneal exudate cells (PEC)) were harvested from B10.PL mice 24 h after intraperitoneal injection with 1 ml of 3% (w/v) thioglycollate. PEC were cultured with media alone for 72 h, then activated with IPN-gamma (100 U ml-1) or treated with media alone. PEC were 98% (w/v) CD11b+ by FACS analysis.

*3,4-DAA treatment.* 3,4-DAA (Angiogen Pharmaceuticals, Pty. Ltd.) was brought into suspension in sodium carboxymethylcellulose (Na-CMC, 0.5%). 3,4-DAA was administered orally in 0,5 ml Na-CMC twice daily using 20-mm feeding needles (Popper and Sons Inc.).

*Induction of experimental autoimmune encephalomyelitis,* EAE was induced in SJL/J mice by subcutaneous immunization with 100 µg of PLP p139-151 emulsified in complete Freund's adjuvant (CPA) containing 4 mg m1-1 of heat-killed Mycobacterium tuberculosis H37Ra (Difco Laboratories). Mice were examined daily for clinical signs of EAE and scored as follows: 0, no paralysis; 1, loss of tail tone; 2, hindlimb weakness; 3, hindlimb paralysis; 4, hindlimb and forelimb paralysis; 5, moribund or dead.

*Flow cytometry.* Immunofluorescent staining was done as described. After incubation for 48 h, cells were washed with FACS buffer (PBS containing 0.1% (w/v) sodium azide and 2% (v/v) FCS) and preincubated with anti-mouse CD16/CD32 monoclonal antibody (clone 2.4G2, PharMingen) for 10 min at 4 °C to block non-speoifc binding to Fc receptors. Fluorochrome-conjugated monoclonal antibodies (rat anti-mouse Mac-1/CD11b-PE (M1/70, IgG2b), mouse anti-mouse MHC class II (I-Ak)-FITC (10-3.6, IgG2b), hamster anti-mouse CD40-FITC (HM40-3, IgM), hamster anti-mouse CD80-FITC (16-10A1, IgG), rat anti-mouse CD86-FITC (GL1, IgG2a), anti-CD4-FITC, anti-CD4-PE, anti-CD44-PE, anti-CD25-FITC and anti-CD59-PE were purchased from PharMingen. Background fluorescence was evaluated by staining the cells with corresponding isotype control antibodies (PharMingen). After incubation, cells were washed twice with FACS buffer and analyzed by FACScan using CellQuest software (Becton Dickinson). For cell cycle analysis splenocytes were washed and stained with anti-CD4-FITC (Phanningen). After fixing the cells with 90% ice-cold ethanol cells were stained with propidium iodide (50 g/ml) in PBS containing 100 U/ml RNase A for 30 min.

*T-cell proliferation assays.* Splenocytes or lymph node cells (LNC) were isolated from mice with EAE and cultured in vitro with the specific encephalitogenic peptide (PLP p139-151) used for the immunization or with concanavalin A (Con A) (positive control) or ovalbumin (negative control). Cells were cultured in 96-well microtitre plates at a concentration of 2-5 times 10⁶ cells ml-1. Culture medium consisted of RPMI 1640 supplemented with L-glutamine (2 mM), sodium pyruvate (1 mM), non-essential amino acids (0.1 mM), penicillin (100 U ml-1), streptomycin (0.1 mg ml-1), 2-mercaptoethanol (5 times 10-5 M) and 10% (v/v) FBS. Splenocytes and LNC from SJL/J mice were incubated for 72 h whereas cultures from MBPAc-1-11 Tg mice were incubated for 48 h. Cultures wore then pulsed for 18 h with 1 µCi per well of [³H]thymidine before harvesting.

*Cytokine analysis.* Supernatants from splenocytes and LNC cultured in parallel with those cells used in proliferation assays tested were used for cytokine analysis. Supernatants were collected at different times for measurements of cytokine levels: 48 h for IL-2 and IL-12/23 p40 and IL-6, 72 h for IFN-γ and TNF-α, and 120 h for IL-4 and IL-10. Cytokine levels were determined by using specific enzyme-linked immunosorbent assay (ELISA) kits for the corresponding cytokines according to the manufacturer's protocols (anti-mouse OPTEIA Kits, PharMingon).

*Semiquantitative PCR.* Mice were sacrifice 60 days after immunization and perfused with 20 ml of cold sterile PBS. Total RNA from spinal cord tissues or microglial cell cultures was isolated using the Absolutely RNA Mini Kit (Stratagene) according to the manufacturer's protocol including an on-column DNA-digestion step. 3 µg of total RNA was converted to cDNA using SuperScript II RNase H- Reverse Transcriptase (Invitrogen, Carlsbad, CA) for first-strand cDNA synthesis. The cDNA product was used for real-time quantitative PCR using a high-speed thermal cycler (LightCycler3; Roche Diagnostics, Indianapolis, IN) and detection of product by SYBR Green I (Qiagen). PCR primers are listed in Suppl. Table 1. The amplification cycles were: 95°C for 900 s, 60 cycles of 94°C for 15 s, 56°C for 20 s, 72°C for 15 s; 6S°C for 15 s, and 40°C for 30 s. β-actin was amplified from all samples as a housekeeping gene to normalize expression. A control (no reverse transcription) was included for each primer set to control for DNA contamination. Melting curves confirmed that only one product was amplified. For quantification, a tenfold dilution series of concentrated total cDNA was included in each reaction.

*iNOS acitvity.* iNOS activity was assessed by the Griess assay as previously described (Platten et al., Biochem Pharmacol 66:1263-70, 2003). Briefly, conditioned supernatant was incubated with an equal volume of Griess reagent containing 1% sulphanilamide, 0.1% naphthylethylenediamine dihydrochloride and 2.5% H3PO4 for 5 min at room temperature. The absorbance was measured at 546 nm. NaNO2 diluted in DMEM served as a standard. To control for cell number, the cells were stained with crystal violet. iNOS activity is expressed as nitrite accumulated in 48 hr/10⁵ cells.

*Western blot analyses.* Microglial cells were lysed in protein extraction buffer containing 20 mg ml-1 aprotinin, 20 mg ml-1 leupeptin, 1.6 mM Pefablook SC (Roche), 10 mM NaF, 1 mM Na3VO4 and 1 mM Na4P2O7 (Sigma), Lysatos were added to 2x SDS loading buffer (Cell Signaling Technology) with 40 mM DTT. Products were separated by electrophoresis on a 10% SDS-PAGE gel. Gels were blotted to PVDF membranes at 100 V in 25 mM Tris, 192 mM glycine and 20% (v/v) methanol, then blocked for 1 h at room temperature with Tris-bufferod saline (TBS) containing 0.1% (v/v) Tween-20 and 5% (wlv) non-fat dry milk. After washing in TBS and 0.1% (v/v) Tween 20, membranes were hybridized overnight at 4 °C with anti-phospho-STAT1α antibody or anti-phosphe-STAT3 antibody (Cell Signaling Technology, Inc.) diluted 1:1,000 in TBS, 0.1% (v/v) Tween 20 and 5% (w/v) BSA. The membranes were then processed by ECL Plus protocol (Amersham BioSciences, Inc.) for visualization of the bands. Membranes were reprobed with anti- anti-STAT-1α as a control to verify equal protein loading. STAT molecules migrated at a relative molecular mass of 90 kDa.

*Histopathology* Anaesthetized mice were perfused with 20 ml cold PBS. Brains and spinal cords were fixed in 4% (w/v) paraformaldehyde and embedded in paraffin. Sections were stained with haematoxylin and eosin. Selected brain, thoracic and lumbar spinal cord sections were evaluated by an examiner blinded to the treatment status of the animal.

*Statistical analysis.* Data are presented as mean and s.e.m. For clinical scores, significance between each two groups was examined by using a one-way multiple-range analysis of variance test (ANOVA) for multiple comparison. A value of p < 0.05 was considered significant.

Those skilled in the art will appreciate that the invention described herein is susceptible to variations and modifications other than those specifically described. It is to be understood that the invention includes all such variations and modifications. The invention also includes all of the steps, features, compositions and compounds referred to or indicated in this specification, individually or collectively, and any and all combinations of any two or more of said steps or features.

### BIBLIOGRAPHY

Bettelli E. et al., J Exp Med 200, 79-87 (Jul 5, 2004).
Brocke, S, et al., Nature 379, 343-6 (1996)
Brown, *et al.,* 1991
Carenno B.M., Collins M., Annu Rev Immunol 20, 29-53 (2002),
Charng M.J. et al., J Food Drug Anal 10, 135-8 (2002).
Garren, H., et al., Immunity 15, 15-22 (2001)
Grohmann U. et al., J Exp Med 198, 153-60 (Jul 7, 2003).
Hardardottir, F., Baron, J.L. & Janeway, C.A., Jr. T cells with two functional antigen-specific receptors. Proc Natl Acad Sci USA 92, 354-8 (1995)
Isaji M., Miyata H., Ajisawa Y., Cardiovascular Drug Reviews 16, 288-299, (1998).
Izawa A., Suzuki J., Takahashi W., Amano J., Isobe M., Artertoscler Thromb Vasc Biol 21, 1172-8. (2001),
Nishibori T., Tanabe Y., Su L., David M., J Exp Med 199, 25-34 (Jan 5, 2004).
Pearson C,I., van Ewijk W., McDevitt H.O., J Exp Med 185, 583-99 (Feb 17,1997).
Platten, M., Eitel, K., Wischhusen, J., Dichgans, J. & Weller, M. Involvement of protein kinase Cdelta and extracellular signal-regulated kinase-2 in the suppression of microglial inducible nitric oxide synthase expression by N-[3,4-dimethoxycinnamoyl]-anthranilic acid (tranilast). Biochem Pharmacol 66, 1263-70 (2003)
Schwarcz R, Curr Opin Pharmacol 4, 12-7 (Feb, 2004).
Steinman, L., Science 305, 212-6 (2004)
Steinmam L., J Exp Med 197, 1065-71 (May 5, 2003).
Steinman L., Nat Immunol 2, 762-4. (2001).
Walker, W.S., Gatewood, J., Olivas, E., Askew, D. & Havenith, C.E. Mouse microglial cell lines differing in constitutive and interferon-gamma-inducible antigen-presenting activities for naive and memory CD4+ and CD8+ T cells, J Neuroimmunol 63, 163-74 (1995)
Wang J., Schreiber R.D., Campbell I.L., Proc Natl Acad Sci U S A 99, 16209-14 (Dec 10, 2002).
Youssef, S. et al. The HMG-CoA reductase inhibitor, atorvastatin, promotes a Th2 bias and reverses paralysis in central nervous system autoimmune disease. Nature 420, 78-84 (2002)

### THE FOLLOWING PARAGRAPHGRAPHS DESCRIBE THE INVENTION

1. A method of down-regulating T_{H}1 cell functioning in a mammal, said method comprising administering to said mammal an effective amount of one or more IDO-mediated tryptophan metabolites or derivatives thereof.
2. The method according to paragraph 1, wherein said administering to said mammal an effective amount of one or more IDO-mediated tryptophan metabolites or derivatives thereof is performed for a time and under conditions sufficient to skew a T_{H}1 cell response to a T_{H}2 cell response.
3. The method according to paragraph 2, wherein said metabolite or derivative thereof up-regulates T_{H}2 cytokine production.
4. The method according to paragraphs 3, wherein said method down-regulates autoimmune T_{H}1 cell functioning, which autoimmune T_{H}1 cell is directed to a myelin protein.
5. The method of any one of paragraphs 1-4, wherein said IDO-mediated tryptophan metabolite or derivative thereof is a compound of formula (I): 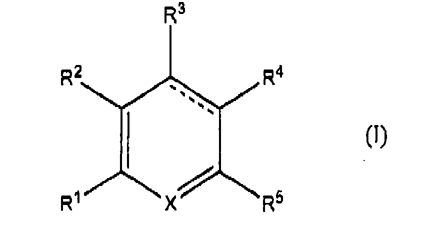 wherein
   X is selected from N and CR⁶;
   ----- represents a single or double bond;
   R¹ is selected from H, C₁₋₄ alkyl, OH, C₁₋₄ alkoxy, halo, CO₂H and CO₂C₁₋₄ alkyl;
   R² is selceted from H, C₁₋₄ alkyl, OH, C₁₋₄ alkoxy, halo, or R¹ and R² together form an optionally substituted fused phenyl ring;
   R³ is selceted from H, C₁₋₄ alkyl, OH, C₁₋₄ alkoxy and halo;
   R⁴ is selected from II, C₁₋₄ alkyl, C₂₋₄ alkenyl, OH, C₁₋₄ alkoxy, CO₂H, CO₂C₁₋₄ alkyl and 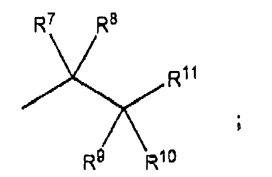 R⁵ is selected from C₁₋₄ alkyl, OH, C₁₋₄ alkoxy, halo, CO₂H, CO₂C₁₋₄ alkyl, NH₂ and NHR¹²;
   R⁶ is selected from II, C₁₋₄ alkyl, OH and C₁₋₄ alkoxy;
   R⁷, R⁸, R⁹ and R¹⁰ are each independently H and C₁₋₄ alkyl or R⁷ and R⁸ together form an oxo group or R⁷ and R⁹ form a bond;
   R¹¹ is selceted from CH(CO₂H)NH₂, CH(CO₂C₁₋₄ alkyl)NH₂, C(O)CO₂H, C(O)CO₂C₁₋₄ alkyl, C(O)H, CO₂H, CO₂C₁₋₄ alkyl, C(O)NH₂, C(O)NHR¹³, CH₂NH₂, CH₂NHC₁₋₄ alkyl and CH₂N(C₁₋₄alkyl)_{2;}
   R¹² is selected from H, C₁₋₄ alkyl and C(O)H; and
   R¹³ is H, C₁₋₄ alkyl and optionally substituted phenyl, wherein optionally substituted phenyl is optionally substituted with one or more, C₁₋₄ alkyl, OH, C₁₋₄ alkoxy, CO₂H, CO₂C₁₋₄ alkyl, halo, NH₂, NHC₁₋₄ alkyl and N(C₁₋₄ alkyl)₂.
6. The method according to paragraph 5, wherein said IDO-mediated tryptophan metabolite is chosen from 3-hydroxykynuronic acid (3-HKA), 3-hydroxyanthranilie acid (3-HAA), picolinic acid (PA) or quinolinic acid (QA).
7. The method of any one of paragraphs 1-4, wherein said IDO-mediated tryptophan metabolite or derivative thereof is a compound of formula (II): 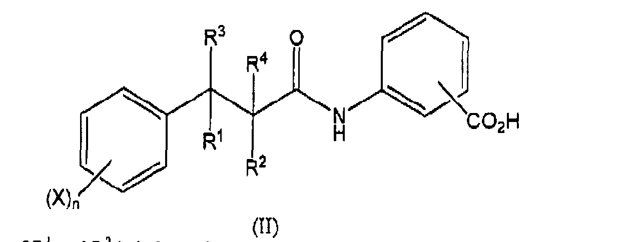 wherein each of R¹ and R² is independently selected from a hydrogen atom or a C₁-C₄ alkyl group, R³ and R⁴ are each hydrogen atoms or together form another chemical bond, each X is independently selected from a hydroxyl group, a halogen atom, a C₁-C₄ alkyl group or a C₁-C₄ alkoxy group, or when two X groups arc alkyl or alkoxy groups, they may be connected together to form a ring, and n is an integer from 1 to 3.
8. The method according to paragraph 7, wherein said CO₂H group is present in the 2-, 3- or 4-position of the aromatic ring.
9. The method according to paragraph 8, wherein CO₂H is in the 2-position.
10. The method according to paragraph 7, wherein at least one of R¹ and R² is a hydrogen atom.
11. The method according to paragraph 10, wherein both of R¹ and R² are hydrogen atoms.
12. The method according to paragraph 7, wherein R³ and R⁴ taken together form a chemical bond.
13. The method according to paragraph 11, wherein said chemical bond is an unsaturated bond in the form of an E or Z geometric isomer.
14. The method according to paragraph 7, wherein n is 1 or 2; each X is the same or different and is selected from halogen, C₁-C₄ alkyl or C₁-C₄ alkoxy.
15. The method according to paragraph 14, wherein X is selected from halogen and C₁-C₄ alkoxy.
16. The method according to paragraph 15, wherein n is 2 and both X arc selected from C₁-C₄ alkoxy
17. The method according to paragraph 16, wherein both X are methoxy.
18. The method of any one of paragraphs 1-4, wherein said IDO-mediated tryptophan metabolite or derivative thereof is a compound chosen from 2-[[3-(2-methylphenyl)-1-oxo-2-propenyl]amino]benzoie acid; 2-[[3-(3-methylphenyl)-1-oxo-2-propenyl]amino]benzoic acid; 2-[[3-(4-methylphenyl)-1-oxo-2-propenyl]amino]benzoic acid; 2-[[3-(2-ethylphenyl)-1-oxo-2-propenyl]amino]benzoie acid; 2-[[3-(3-ethylphenyl)-1-oxo-2-propenyl]amino]benzoic acid; 2-[[3-(4-ethylphenyl)-1-oxo-2-propenyl]amino]benzoic acid; 2-[[3-(2-propylphenyl)-1-oxo-2-propenyl]amino]benzoic acid; 2-[[3-(3-propylphenyl)-1-oxo-2-propenyl]amino]benzoic acid; 2-[[3-(4-propylphenyl)-1-oxo-2-propenyl]amino]benzoic acid; 2-[[3-(2-hydroxyphenyl)-1-oxo-2-propenyl]amino]benzoic acid; 2-[[3-(3-hydroxyphenyl)-1-oxo-2-propenyl]amino]benzoic acid; 2-[[3-(4-hydroxyphenyl)-1-oxo-2-propenyl]amino]benzic acid; 2-[[3-(2-chlorophenyl)-1-oxo-2-propenyl]amino]benzoic acid; 2-[[3-(3-chlorophenyl)-1-oxo-2-propenyl]amino]benzoic acid; 2-[[3-(4-chlorophenyl)-1-oxo-2-propenyl]amino]benzoic acid; 2-[[3-(2-fluorophenyl)-1-oxo-2-propenyl]amino]benzoic acid; 2-[[3-(3-flurophenyl)-1-oxo-2-propenyl]amino]benzoic acid; 2-[[3-(4-fluorophenyl)-1-oxo-2-propenyl]amino]benzioc acid; 2-[[3-(2-bromophenyl)-1-oxo-2-propenyl]amino]benzoic acid; 2-[[3-(3-bromophenyl)-1-oxo-2-prepenyl]amino]benzoic acid; 2-[[3-(4-bromophenyl)-1-oxo-2-propenyl]amino]benzoic acid; 2-[[3-(2,3-dimethoxyphenyl)-1-oxo-2-propenyl]amino]benzoic acid; 2-[[3-(3,4-dimethoxyphenyl)-1-oxo-2-propenyl]amino]benzoic acid; 2-[[3-(2,4-dimethoxyphenyl)-1-oxo-2-prospenyl]amino]benzeic acid; 2-[[3-(2,3-dimethylphenyl)-1-oxo-2-propenyl]amino]benzoic acid; 2-[[3-(3,4-dimethylphenyl)-1-oxo-2-propenyl]amino]benzoic acid; 2-[[3-(2,4-dimethylphenyl)-1-oxo-2-propenyl]amino]benzoic acid; 2-[[3-(2,3-diethoxyphenyl)-1-oxo-2-propenyl]amino]benzoic acid; 2-[[3-(3,4-diethoxyphenyl)-1-oxo-2-propenyl]amino]benzoic acid; 2-[[3-(2,4-diethoxyphenyl)-1-oxo-2-propenyl]amino]benzoic acid; 2-[[3-(2,3-dipropoxyphenyl)-1-oxo-2-propenyl]amino]benzoic acid; 2-[[3-(3,4-dipropoxyphenyl)-1-oxo-2-propenyl]amino]benzoic acid; 2-[[3-(2,4-dipropoxyphenyl)-1-oxo-2-propenyl]amino]benzolc acid; 2-[[3-(2,3-dlethylphenyl)-1-oxo-2-propenyl]amino]benzolc acid; 2-[[3-(3,4-diethylphenyl)-1-oxo-2-propenyl]amino]benzoic acid; 2-[[3-(2,4-diethylphenyl)-1-oxo-2-propenyl]amino]benzoic acid; 2-[[3-(2,3-dipropylphenyl)-1-oxo-2-propenyl]amino]benzoic acid; 2-[[3-(3,4-dipropylphenyl)-1-oxo-2-propenyl]amino]benzoic acid; 2-[[3-(2,4-dipropylphenyl)-1-oxo-2-propenyl]amino]benzoic acid; 2-[[3-(2-methoxy-3-methylphenyl)-1-oxo-2-propenyl]amino]benzoic acid; 2-[[3-(3-methoxy-4-methylphenyl)-1-oxo-2-propenyl]amino]benzoic acid; 2-[[3-(2-methoxy-3-methylphenyl)-1-oxo-2-propenyl]-amino]benzoic acid; 2-[[3-(2-methoxy-4-methylphenyl)-1-oxo-2-propenyl]amino]benzoic acid; 2-[[3-(2-metboxy-3-chlorophenyl)-1-oxo-2-propenyl]amino]benzoic acid; 2-[[3-(3-methoxy-4-chlorophenyl)-1-oxo-2-propenyl]amino]benzoic acid; 2-[[3-(2-methoxy-3-chlorophenyl)-1-oxo-2-propenyl]amino]benzoic acid; 2-[[3-(2-methoxy-4-chlorophenyl)-1-oxo-2-propenyl]amino]benzoic acid; 2-[[3-(2-methoxy-3-hydroxyphenyl)-1-oxo-2-propenyl]-amino]benzoic acid; 2-[[3-(3-methoxy-4-hydroxyphenyl)-1-oxo-2-propenyl]amino]benzoic acid; 2-[[3-(2-methoxy-3-hydroxyphenyl)-1-oxo-2-propeny]amino]benzoic acid; 2-[[3-(2-methoxy-4-hydroxyphenyl)-1-oxo-2-propenyl]amino]benzoic acid; 2-[[3-(3,4-trimethylene-phenyl)-1-oxo-2-propenyl]amino]benzoic acid; 2-[[3-(2,3-trimethylenephenyl)-1-oxo-2-propenyl]amino]benzoic acid; 2-[[3-(3,4-methylenedioxyphenyl)-1-oxo-2-propenyl]amino]-benzoic acid; and 2-[[3-(3,4-ethylenedioxyphenyl)-1-oxo-2-propenyl]amino]benzoic acid.
19. The method of any one of paragraphs 1-4, wherein said IDO-mediated tryptophan metabolite or derivative thereof is 2-[[3-(3,4-dimethoxyphenyl)-1-oxo-2-propenyl]amino]benzoic acid (tranilast, TNL).
20. The method according to any one of paragraphs 1-19, further comprising administering an agonistic agent in order to enhance the effects of said IDO-mediated tryptophan metabolite or derivative thereof.
21. A composition for use in any of the methods of paragraphs 1-20.
22. The use of an IDO-mediated tryptophan metabolite or derivative thereof in the manufacture of a medicament for a method according to any one of paragraphs 1-20.
23. A pharmaceutical composition, comprising an IDO-mediated tryptophan metabolite or derivative thereof; and a pharmaceutically acceptable excipient.
24. The pharmaceutical composition of paragraph 23, wherein said IDO-mediated tryptophan metabolite or derivative thereof is a compound of formula (I): 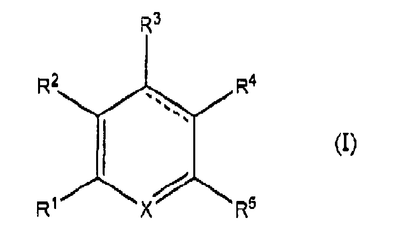 wherein
   X is selected from N and CR⁶;
   ----- represents a single or double bond;
   R¹ is selected from H, C₁₋₄ alkyl, OH, C₁₋₄ alkoxy, halo, CO₂H and CO₂C₁₋₄ alkyl;
   R² is selected from H, C₁₋₄ alkyl, OH, C₁₋₄ alkoxy, halo, or R¹ and R² together form an optionally substituted fused phenyl ring;
   R³ is selected from H, C₁₋₄ alkyl, OH, C₁₋₄ alkoxy and halo;
   R⁴ is selected from H, C₁₋₄ alkyl, C₂₋₄ alkenyl, OH, C₁₋₄ alkoxy, CO₂H, CO₂C₁₋₄ alkyl and 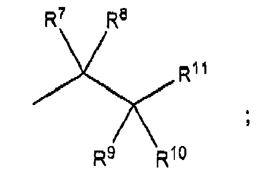 R⁵ is selected from C₁₋₄ alkyl, OH, C₁₋₄ alkoxy, halo, CO₂H, CO₂C₁₋₄ alkyl, NH₂ and NHR¹²; R⁶ is selected from H, C₁₋₄ alkyl, OH and C₁₋₄ alkoxy;
   R⁷, R⁸, R⁹ and R¹⁰ are each independently H and C₁₋₄ alkyl or R⁷ and R⁸ together form an oxo group or R⁷ and R⁹ form a bond;
   R¹¹ is selected from CH(CO₂H)NH₂, CH(CO₂C₁₋₄ alkyl)NH₂, C(O)CO₂H, C(O)CO₂C₁₋₄ alkyl, C(O)H, CO₂H, CO₂C₁₋₄ alkyl, C(O)NH₂, C(O)NHR¹³, CH₂NH₂, CH₂NHC₁₋₄ alkyl and CH₂N(C₁₋₄ alkyl)₂;
   R¹² is selected from II, C₁₋₄ alkyl and C(O)H; and
   R¹³ is H, C₁₋₄ alkyl and optionally substituted phenyl, wherein optionally substituted phenyl is optionally substituted with one or more, C₁₋₄ alkyl, OH, C₁₋₄ alkoxy, CO₂H, CO₂C₁₋₄ alkyl, halo, NH₂, NHC₁₋₄ alkyl and N(C₁₋₄ alkyl)₂.
25. The pharmaceutical composition of paragraph 23, wherein said IDO-mediated tryptophan metabolite is chosen from 3-hydroxykynurenie acid (3-HKA), 3-hydroxyanthranilic acid (3-HAA), picolinic acid (PA) or quinolinic acid (QA).
26. The pharmaceutical composition of paragraph 23, wherein said IDO-mediated tryptophan metabolite or derivative thereof is a compound of formula (II): 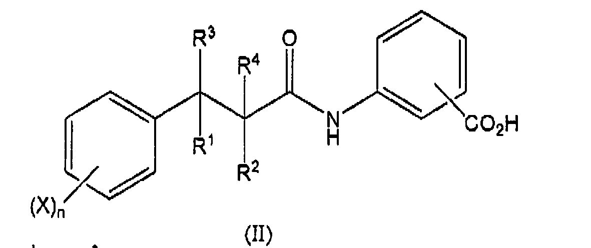 wherein each of R¹ and R² is independently selected from a hydrogen atom or a C₁-C₄ alkyl group, R³ and R⁴ are each hydrogen atoms or together form another chemical bond, each X is independently selected from a hydroxyl group, a halogen atom, a C₁-C₄ alkyl group or a C₁-C₄ alkoxy group, or when two X groups are alkyl or alkoxy groups, they may be connected together to form a ring, and n is an integer from 1 to 3.
27. The pharmaceutical composition of paragraph 26, wherein said CO₂H group is present in the 2-, 3- or 4-position of the aromatic ring.
28. The pharmaceutical composition of paragraph 27, wherein CO₂H is in the 2-position.
29. The pharmaceutical composition of paragraph 26, wherein at least one of R¹ and R² is a hydrogen atom.
30. The pharmaceutical composition of paragraph 26, wherein both of R¹ and R² are hydrogen atoms.
31. The pharmaceutical composition of paragraph 26, wherein R³ and R⁴ taken together form a chemical bond.
32. The pharmaceutical composition of paragraph 31, wherein said chemical bond is an unsaturated bond in the form of an E or Z geometric isomer.
33. The pharmaceutical composition of paragraph 26, wherein n is 1 or 2; each X is the same or different and is selected from halogen, C₁-C₄ alkyl or C₁-C₄ alkoxy.
34. The pharmaceutical composition of paragraph 26, wherein X is selected from halogen and C₁-C₄ alkoxy.
35. The pharmaceutical composition of paragraph 26, wherein n is 2 and both X are selected from C₁-C₄ alkoxy
36. The pharmaceutical composition of paragraph 26, wherein both X arc methoxy.
37. The pharmaceutical composition of paragraph 26, wherein said IDO-mediated tryptophan metabolite or derivative thereof is a compound chosen from 2-[[3-(2-methylphenyl)-1-oxo-2-propenyl]amino]benzoic acid; 2-[[3-(3-methylphenyl)-1-oxo-2-propenyl]amino]benzoic acid; 2-[[3-(4-methylphenyl)-1-oxo-2-propenyl]amino]benzoic acid; 2-[[3-(2-ethylphenyl)-1-oxo-2-propenyl]amino]benzoic acid; 2-[[3-(3-ethylphenyl)-1-oxo-w-propenyl]amino]benzoic acid; 2-[[3-(4-ethylphenyl)-1-oxo-2-propenyl]amino]benzoic acid; 2-[[3-(2-propylphenyl)-1-oxo-2-propenyl]amino]benzoic acid; 2-[[3-(3-propylphenyl)-1-oxo-2-propenyl]amino]benzoic acid; 2-[[3-(4-propylphenyl)-1-oxo-2-propenyl]amino]benzoic acid; 2-[[3-(2-hydroxyphenyl)-1-oxo-2-propenyl]amino]benzoic acid; 2-[[3-(3-hydroxyphenyl)-1-oxo-2-propenyl]amino]benzoic acid; 2-[[3-(4-hydroxyphenyl)-1-oxo-2-propenyl]-amino]benzoic acid; 2-[[3-(2-chlorophenyl)-1-oxo-2-propenyl]amino]benzoic acid; 2-[[3-(3-chlorophenyl)-1-oxo-2-propenyl]amino]benzoic acid; 2-[[3-(4-chlorophenyl)-1-oxo-2-propenyl]amino]benzoic acid; 2-[[3-(2-fluorophenyl)-1-oxo-2-propenyl]amino]benzoic acid; 2-[[3-(3-fluorophenyl)-1-oxo-2-propenyl]amino]benzoic acid; 2-[[3-(4-fluorophenyl)-1-oxo-2-propenyl]amino]benzoic acid; 2-[[3-(2-bromophenyl)-1-oxo-2-propenyl]amino]benzoic acid; 2-[[3-(3-bromophenyl)-1-oxo-2-propenyl]amino]benzoic acid; 2-[[3-(4-bromophenyl)-1-oxo-2-propenyl]amino]benzoic acid; 2-[[3-(2,3-dimethoxyphenyl)-1-oxo-2-propenyl]-amino]benzoic acid; 2-[[3-(3,4-dimethoxyphenyl)-1-oxo-2-propenyl]amino]benzoic acid; 2-[[3-(2,4-dimethoxyphenyl)-1-oxo-2-propenyl]amino]benzoic acid; 2-[[3-(2,3-dimethylphenyl)-1-oxo-2-propenyl]amino]benzoic acid; 2-[[3-(3,4-dimethylphenyl)-1-oxo-2-propenyl]amino]benzoic acid; 2-[[3-(2,4-dimethylphenyl)-1-oxo-2-propenyl]amino]benzoic acid; 2-[[3-(2,3-diethoxyphenyl)-1-oxo-2-propenyl]amino]benzoic acid; 2-[[3-(3,4-diethoxyphenyl)-1-oxo-2-propenyl]amino]benzoic acid; 2-[[3-(2,4-diethoxyphenyl)-1-oxo-2-propenyl]amino]benzoic acid; 2-[[3-(2,3-dipropoxyphenyl)-1-oxo-2-propenyl]amino]benzoic acid; 2-[[3-(3,4-dipropoxyphenyl)-1-oxo-2-propenyl]amino]benzoic acid; 2-[[3-(2,4-dipropoxyphenyl)-1-oxo-2-propenyl]amino]benzoic acid; 2-[[3-(2,3-diethylphenyl)-1-oxo-2-propenyl]amino]benzoic acid; 2-[[3-(3,4-diethylphenyl)-1-oxo-2-propenyl]amino]benzoic acid; 2-[[3-(2,4-diethylphenyl)-1-oxo-2-propenyl]amino]amino]benzoic acid; 2-[[3-(2,3-dipropyl-phenyl)-1-oxo-2-propenyl]amino]benzoic acid; 2-[[3-(3,4-dipropylphenyl)-1-oxo-2-propenyl]amino]benzoic acid; 2-[[3-(2,4-dipropylphenyl)-1-oxo-2-propenyl]amino]benzoic acid; 2-[[3-(2-methoxy-3-methylpheny)-1-oxo-2-propenyl]amino]benzoic acid; 2-[[3-(3-methoxy-4-methylphenyl)-1-oxo-2-propeny]amino]benzoic acid; 2-[[3-(2-methoxy-3-methylphenyl)-1-oxo-2-propenyl]amino]benzoic acid; 2-[[3-(2-methoxy-4-methylphenyl)-1-oxo-2-propenyl]amino]benzoic acid; 2-[[3-(2-methoxy-3-chlorophenyl)-1-oxo-2-propenyl]-amino]benzoic acid; 2-[[3-(3-methoxy-4-chlorophenyl)-1-oxo-2-propenyl]amino]benzoic acid; 2-[[3-(2-methoxy-3-chlorophenyl)-1-oxo-2-propenyl]amino]benzoic acid; 2-[[3-(2-methoxy-4-chlorophenyl)-1-oxo-2-propenyl]amino]benzoic acid; 2-[[3-(2-methoxy-3-hydroxyphenyl)-1-oxo-2-propenyl]amino]benzoic acid; 2-[[3-(3-methoxy-4-hydroxyphenyl)-1-oxo-2-propenyl]amino]benioic acid; 2-[[3-(2-methoxy-3-hydroxyphenyl)-1-oxo-2-propenyl]amino]benzoic acid; 2-[[3-(2-methoxy-4-hydroxyphenyl)-1-oxo-2-propenyl]amino]-benzoic acid; 2-[[3-(3,4-trimethylenephenyl)-1-oxo-2-propenyl]amino]benzoic acid; 2-[[3-(2,3-trimethylenephenyl)-1-oxo-2-propenyl]amino]benzoic acid; 2-[[3-(3,4-methylenedioxyphenyl)-1-oxo-2-propenyl]amino]benzoic acid; and 2-[[3-(3,4-ethylencdioxyphcnyl)-1-oxo-2-propenyl]amino]benzoic acid.
38. The pharmaceutical composition of paragraph 26, wherein said IDO-mediated tryptophan metabolite or derivative thereof is 2-[[3-(3,4-dimethoxyphenyl)-1-oxo-2-propenyl]amino]benzoic acid (tranilast, TNL).
39. A method of upregulating in a mammal T_{H}1 cell functioning, said method comprising administering to said mammal an effective amount of an antagonist of an IDO-mediated tryptophan metabolite or compound of formula (I) or formula (II) or a pharmaceutically acceptable salt thereof.

## Claims

1. 2-[[3-(3,4-dimethoxyphenyl)-1-oxo-2-propenyl]amino]benzoic acid (tranilast) or a pharmaceutically acceptable salt thereof for use in treating an autoimmune disease by selectively downregulating T_{H}1 cell functioning, wherein said tranilast downregulates T_{H}1 cell functioning.

2. Tranilast or a pharmaceutically acceptable salt thereof for use according to claim 1, wherein said autoimmune disease is an autoimmune demyelinating disease of the central nervous system or periphery.

3. Tranilast or a pharmaceutically acceptable salt thereof for use according to claim 2, wherein said autoimmune demyelinating disease is multiple sclerosis, acute inflammatory polyradiculopathy (Guillan-Barre syndrome), polyradiculopathy or chronic inflammatory demyleniation.

4. Tranilast or a pharmaceutically acceptable salt thereof for use according to claim 3, wherein said autoimmune demyelinating disease is multiple sclerosis.

5. An *in vitro* method of downregulating T cell functioning, said method comprising contacting said T cell with an effective amount of 2-[[3-(3,4-dimethoxyphenyl)-1-oxo-2-propenyl]amino]benzoic acid (tranilast) or a pharmaceutically acceptable salt thereof.


SEQUENCE LISTING

<110> STEINMAN, Lawrence
PLATTEN, Michael
HO, Peggy Pui-Kay

<120> A METHOD OF MODULATING T CELL FUNCTIONING
<130> STAN-473WO

<150> 60/644,502
<151> 2005-01-14

<160> 14
<170> PatentIn version 3.1

<210> 1
<211> 18
<212> DNA
<213> Mus musculus

<400> 1
ggatgcttcc tgagtttg 18

<210> 2
<211> 21
<212> DNA
<213> Mus musculus

<400> 2
acggtcacta cacttaaaat g 21

<210> 3
<211> 16
<212> DNA
<213> Mus musculus

<400> 3
ccaaggtcag cgttcc 16

<210> 4
<211> 21
<212> DNA
<213> Mus musculus

<400> 4
gacgtttatg ttgtagaggt g 21

<210> 5
<211> 16
<212> DNA
<213> Mus musculus

<400> 5

aatgtgcccc gtatcc 16

<210> 6
<211> 25

<212> DNA
<213> Mus musculus


<400> 6
ccttgtctac tgctaaccga ctcct 25


<210> 7
<211> 16
<212> DNA
<213> Mus musculus


<400> 7
gacggcaaac atgact 16


<210> 8
<211> 18
<212> DNA
<213> Mus musculus


<400> 8
ctggtttcat aaacgccg 18


<210> 9
<211> 22
<212> DNA
<213> Mus musculus


<400> 9
cataactata atgctacggg ga 22


<210> 10
<211> 17
<212> DNA
<213> Mus musculus


<400> 10
gtttggtccc gtgtgat 17


<210> 11
<211> 16
<212> DNA
<213> Mus musculus


<400> 11
gtctcgcctc ctttgt 16

<210> 12
<211> 17
<212> DNA
<213> Mus musculus

<400> 12
ggaggtgtga agttgct 17

<210> 13
<211> 19
<212> DNA
<213> Mus musculus

<400> 13
agttggtccc ccttctcca 19

<210> 14
<211> 17
<212> DNA
<213> Mus musculus

<400> 14
ccactcgtac ttgggat 17